# EUROPEAN PATENT APPLICATION

(11) **EP 4 148 059 A1**
(43) Date of publication of application: **15.03.2023**
(21) Application number: 21784026.3
(22) Date of filing: 12.04.2021
(51) Int. Cl.: C07H 1/00, C07H 15/04, A61K 47/54, A61K 49/04

(54) **COMPOUND, CONTRAST MEDIUM, AND METHOD FOR PRODUCING COMPOUND**

(30) Priority: 10.04.2020 JP 2020071252
(71) Applicant: St. Marianna University School of Medicine, Kawasaki-shi Kanagawa 216-8511 (JP); Sophia School Corporation, Tokyo 102-8554 (JP)
(72) Inventor: MATSUMOTO, Nobuyuki, Kawasaki-shi, Kanagawa 216-8511 (JP); SUZUKI, Yumiko, Tokyo 102-8554 (JP)
(74) Representative: Mewburn Ellis LLP
(86) International application number: PCT/JP2021/015216
(87) International publication number: WO 2021/206182

(57) **Abstract**

The present invention relates to a compound represented by the formula (1) or a pharmaceutically acceptable salt thereof. In the formula (1), R¹ to R³ are each independently a predetermined amino group or a predetermined amide group, or a group represented by the formula (2), and at least one of R¹ to R³ is a group represented by the formula (2).

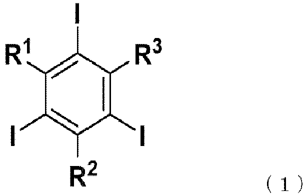

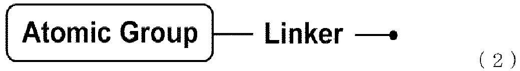

## Description

### Technical Field

The present invention relates to a compound, a contrast agent, and a method for producing a compound.

### Background Art

Contrast radiography using iodine contrast agents largely enhances the diagnostic performance of X-ray images and is a diagnostic method that takes an important position in many diagnosis and treatment departments. Most of iodine contrast agents are excreted from the kidney. Hence, they suffer from the problem of contrast nephropathy that occurs as an adverse reaction.

For example, Patent Literature 1 proposes use of a conjugate of a nonionic iodine contrast agent and a group that is recognized by a hepatocyte-specific transporter, as a method for solving the problem described above. The conjugate serves as a substrate of the transporter and is taken up into hepatocytes or excreted from the hepatocytes. Thus, a portion of the agent that would otherwise be renally excreted can be excreted into bile, thereby alleviating nephrotoxicity. Patent Literature 1 specifically discloses a conjugate having iohexol as the nonionic iodine contrast agent and an ethoxybenzyl group or a group derived from ursodeoxycholic acid as the group that is recognized by a hepatocyte-specific transporter.

### Citation List

### Patent Literature

Patent Literature 1: Japanese Patent Laid-Open No. 2018-062475

### Summary of Invention

### Technical Problem

Iodine contrast agents are suitably administered by intravenous injection. In the case of intravenously administering a high concentration of an injection containing the conjugate of Patent Literature 1, the viscosity of the drug may increase. Hence, the iodine contrast agents are required to be excellent in handleability when administered while having the ability to alleviate nephrotoxicity.

An object of the present invention is to provide a novel compound that can be used as an iodine contrast agent.

### Solution to Problem

The present inventors have conducted diligent studies to attain the object and consequently completed the present invention by finding a novel compound capable of serving as an iodine contrast agent.

Specifically, the present invention is as follows.
[1] A compound represented by the formula (1) or a pharmaceutically acceptable salt thereof: 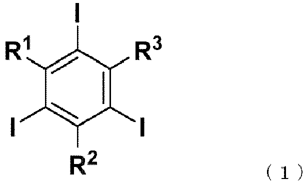 wherein
   R¹ to R³ are each independently
   an amino group represented by -NR^{x}R^{y} wherein R^{x} and R^{y} each independently represent a hydrogen atom, a C1 to C6 hydrocarbon group optionally having a substituent, or a C2 to C7 acyl group optionally having a substituent; or
   an amide group represented by -C(=O)NR^{z}R^{w} wherein R^{z} and R^{w} each independently represent a hydrogen atom or a C1 to C6 hydrocarbon group optionally having a substituent; or
   a group represented by the formula (2): 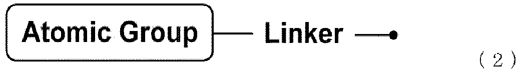 wherein
   Atomic Group is an atomic group that binds to an asialoglycoprotein receptor, and
   Linker is an arbitrary linker, and
   at least one of R¹ to R³ is the group represented by the formula (2).
[2] The compound according to [1] or a pharmaceutically acceptable salt thereof, wherein
   the Atomic Group is a sugar residue that binds to an asialoglycoprotein receptor.
[3] The compound according to [1] or [2] or a pharmaceutically acceptable salt thereof, wherein
   the Atomic Group is a group derived from galactose, N-acetylgalactosamine, N-trifluoroacetylgalactosamine, or galactose-N-acetylglucosamine.
[4] The compound according to any one of [1] to [3] or a pharmaceutically acceptable salt thereof, wherein
   the group represented by the formula (2) is a group represented by the following formula (2-1), (2-2), (2-3), or (2-4): 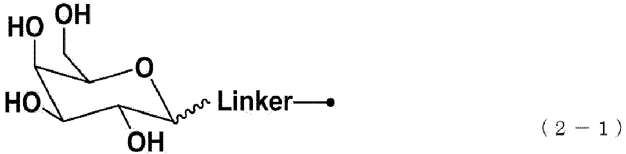 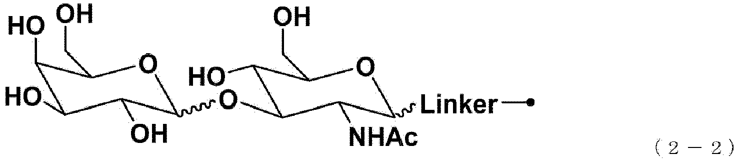 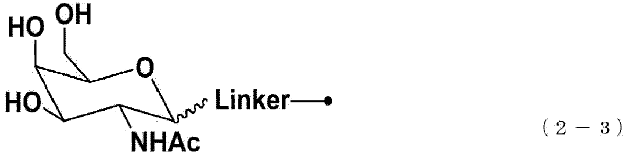 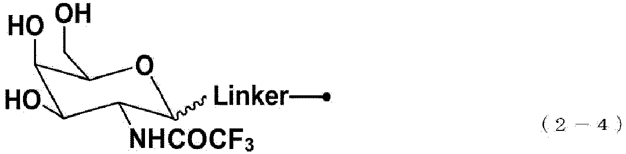 wherein Linker is an arbitrary linker.
[5] The compound according to any one of [1] to [4] or a pharmaceutically acceptable salt thereof, wherein
   the linker is a hydrocarbon chain optionally having a substituent, and
   one or both of the two ends of the hydrocarbon chain optionally have a heteroatom, an amide bond, an ester bond, a carbonyl bond, or an aromatic heterocycle.
[6] The compound according to [5] or a pharmaceutically acceptable salt thereof, wherein
   the hydrocarbon chain is an alkylene chain optionally having a substituent, or a hydrocarbon chain formed by bonding two or more alkylene chains optionally having a substituent via at least one selected from the group consisting of a heteroatom, an amide bond, an ester bond, a carbonyl bond, and an aromatic heterocycle.
[7] The compound according to any one of [1] to [6] or a pharmaceutically acceptable salt thereof, wherein
   the linker is represented by the following structure: 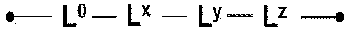 wherein
   L° is -O- or -NHC(=O)-,
   L^{x} is represented by the following structure: 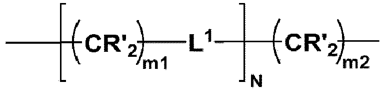
   L^{y} represents a single bond or is represented by the following structure: 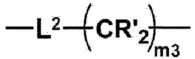 and
   L^{z} is represented by the following structure: 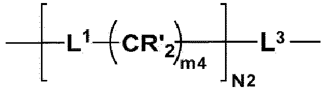 wherein
   each R' is independently one selected from a hydrogen atom, a C1 to C3 alkyl group optionally having a substituent, and a hydroxy group,
   each L¹ is independently one selected from an ether bond (-O-), a thioether bond (-S-), an amine bond (-NH-), an amide bond, an ester bond, and a carbonyl bond,
   L² is an amide bond, or a divalent group derived from an aromatic heterocycle,
   L³ is one selected from -OCH₂-, -NHCH₂-, -C(=O)NH-, - C(=O)NHCH₂-, and -NHC(=O)-,
   m1, m2, and m4 are each independently an integer of 1 or larger,
   m3 is an integer of 0 or larger, and
   N and N2 are each independently an integer of 0 or larger.
[8] The compound according to [7] or a pharmaceutically acceptable salt thereof, wherein
   L^{x} is any of the following structures: 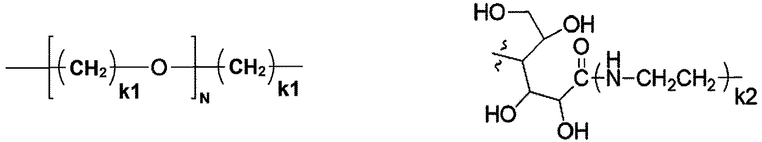
   L^{y} is a single bond or any of the following structures: 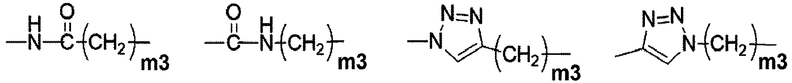 and
   L^{z} is any of the following structures: 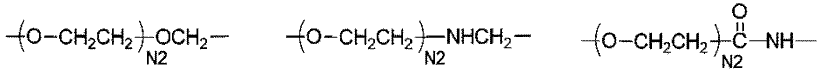 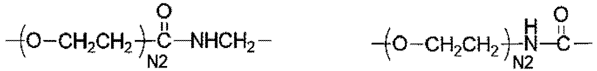 wherein
   each k1 is independently an integer of 1 or larger and 3 or smaller,
   k2 is 0 or 1,
   m3 is an integer of 0 or larger, and
   N and N2 are each independently an integer of 0 or larger.
[9] The compound according to [7] or a pharmaceutically acceptable salt thereof, wherein
   the linker is represented by the following structure: 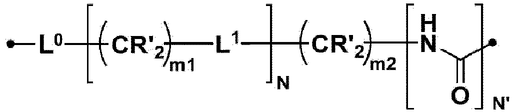 wherein
   each R' is independently one selected from a hydrogen atom, a C1 to C3 alkyl group optionally having a substituent, and a hydroxy group,
   L° is -O- or -NHC(=O)-,
   each L¹ is independently one selected from an ether bond (-O-), a thioether bond (-S-), an amine bond (-NH-), an amide bond, an ester bond, and a carbonyl bond,
   m1 and m2 are each independently an integer of 1 or larger,
   N is an integer of 0 or larger, and
   N' is 0 or 1.
[10] The compound according to [9] or a pharmaceutically acceptable salt thereof, wherein
   the linker is represented by any of the following structures: 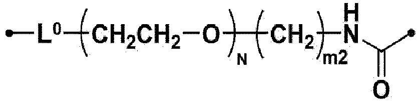 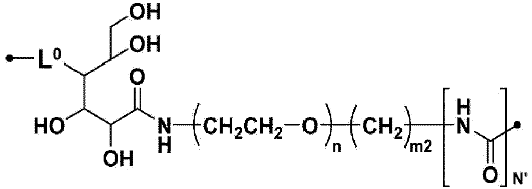 wherein
   L° is -O- or -NHC(=O)-,
   N is an integer of 0 or larger,
   n is an integer of 0 or larger,
   m2 is an integer of 1 or larger, and
   N' is 0 or 1.
[11] The compound according to any one of [1] to [10] or a pharmaceutically acceptable salt thereof, wherein
   the amino group is represented by any of the following structures: 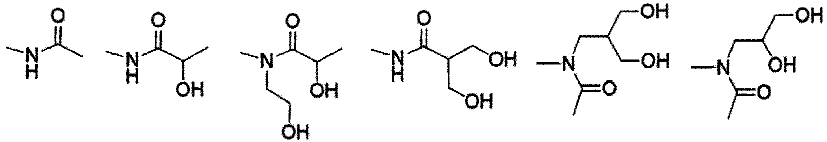 and
   the amide group is represented by any of the following structures: 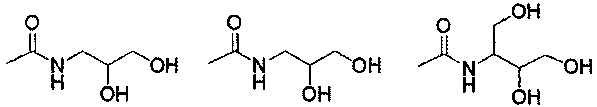
[12] The compound according to any one of [1] to [11] or a pharmaceutically acceptable salt thereof, wherein
   the amino group is an acetylamino group.
[13] The compound according to any one of [1] to [12] or a pharmaceutically acceptable salt thereof, wherein
   in the formula (1), all of R¹ to R³ are groups represented by the formula (2).
[14] A compound represented by any of the following structures or a pharmaceutically acceptable salt thereof: 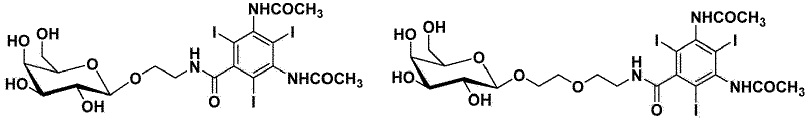 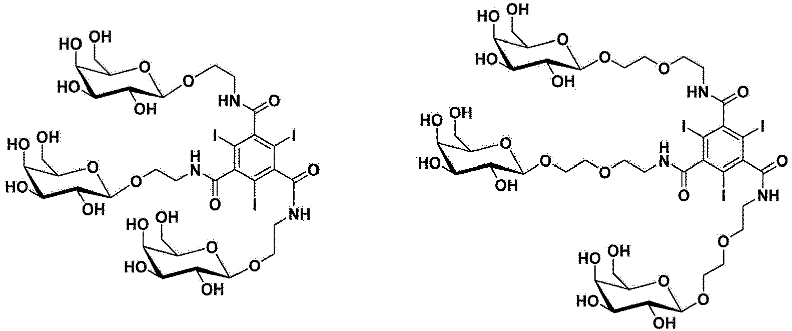 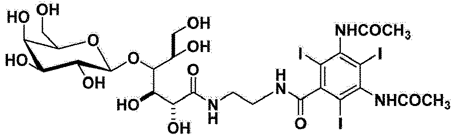 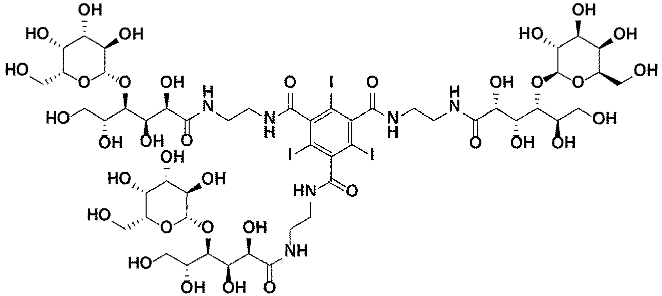
[15] A compound represented by the following structure or a pharmaceutically acceptable salt thereof: 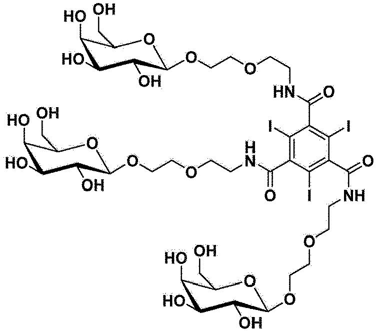
[16] A compound represented by the following structure or a pharmaceutically acceptable salt thereof: 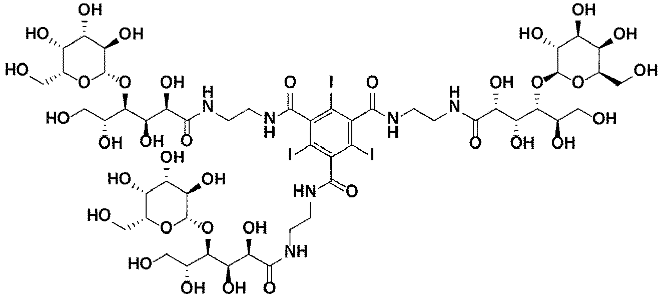
[17] A contrast agent comprising a compound according to any one of [1] to [16] or a pharmaceutically acceptable salt thereof.
[18] A method for producing a compound according to any one of [1] to [16] or a pharmaceutically acceptable salt thereof, comprising the step of
   reacting a reaction substrate constituted by an atomic group moiety that binds to an asialoglycoprotein receptor and a linker moiety with a reaction substrate of a nonionic iodine contrast agent moiety.

### Advantageous Effects of Invention

The present invention can provide a novel compound that can be used as an iodine contrast agent.

### Brief Description of Drawings

[Figure 1] Figure 1 is a diagram showing results of an uptake inhibition test of RYO-1, MEG-1, and MEG-2, which are compounds of Examples, in HepG2.
[Figure 2] Figure 2 is a diagram showing results of an uptake inhibition test of RYO-1, MEG-1, and MEG-2, which are compounds of Examples, in Panc-1.
[Figure 3] Figure 3 is a diagram showing results of an uptake inhibition test of MEG-2, MEG-4, RYO-1, and RYO-2 which are compounds of Examples in HepG2.
[Figure 4] Figure 4 is a diagram showing a CT image taken 1 hour after administration when MEG-1, which is a compound of Example, was administered to a mouse.

### Description of Embodiments

Hereinafter, the mode for carrying out the present invention will be described in detail. However, the present invention is not limited by the following embodiments and can be carried out by making various changes or modifications therein without departing from the spirit of the present invention.

### [Compound]

The compound of the present invention is represented by the formula (1). The present invention also provides a pharmaceutically acceptable salt of the compound represented by the formula (1). In the present specification, both of a compound and a pharmaceutically acceptable salt thereof are simply referred to as a compound.

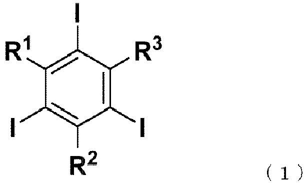

In the formula (1), R¹ to R³ are each independently
an amino group represented by -NR^{x}R^{y} wherein R^{x} and R^{y} each independently represent a hydrogen atom, a C1 to C6 hydrocarbon group optionally having a substituent or a C2 to C7 acyl group optionally having a substituent, or
an amide group represented by -C(=O)NR^{z}R^{w} wherein R^{z} and R^{w} each independently represent a hydrogen atom or a C1 to C6 hydrocarbon group optionally having a substituent, or
a group represented by the formula (2): 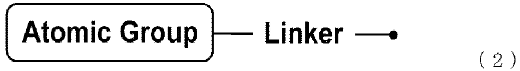 wherein
Atomic Group is an atomic group that binds to an asialoglycoprotein receptor, and
Linker is an arbitrary linker, and
at least one of R¹ to R³ is a group represented by the formula (2).

In the compound of the present invention, when one of R¹ to R³ is a group represented by the formula (2), the remaining two of R¹ to R³ are selected from the amino group and the amide group. When two of R¹ to R³ are groups represented by the formula (2), the remaining one of R¹ to R³ is selected from the amino group and the amide group. The compound of the present invention also includes the case where all of R¹ to R³ are groups represented by the formula (2).

When each of the groups R¹ to R³ in the compound of the present invention comprises a plurality of groups represented by the formula (2), a plurality of the amino groups, or a plurality of the amide groups, these groups may be the same or different.

In the compound of the present invention, respective forms in the description about R¹ to R³ may be arbitrarily combined, regardless of whether to be preferable.

The compound of the present invention can be used as a contrast agent. In the present invention, the contrast agent refers to a medicament or a pharmaceutical composition that is administered to a patient in order to impart a contrast to an image to be taken or emphasize a particular tissue in diagnostic imaging.

The Atomic Group in the compound of the present invention is recognized by an asialoglycoprotein receptor specifically expressed in a hepatocyte so that the compound is taken up into the hepatocyte. Most of existing iodine contrast agents are wholly excreted into urine from the kidney and thus place burdens on the kidney, whereas the compound of the present invention can be taken up into the liver and excreted into bile. Specifically, the compound of the present invention can employ urine and bile as two systems of drug metabolism pathways and can alleviate nephrotoxicity.

Furthermore, the compound of the present invention, when administered in the form of an injection, tends to be able to maintain constant viscosity even if the compound has a high concentration. Thus, the compound of the present invention is excellent in handleability and can be easily administered to a recipient.

The compound represented by the formula (1) of the present invention can be represented by, for example, the formula (1-1), (1-2), (1-3), (1-4), (1-5), or (1-6) given below. The compound represented by the formula (1) of the present invention preferably has two or more groups represented by the formula (2) and more preferably has three groups represented by the formula (2). Specifically, the compound of the present invention is preferably represented by the formula (1-2), (1-3), or (1-5) given below and more preferably represented by the formula (1-3) given below. According to such an aspect, the interaction between the compound of the present invention and a substrate recognition site (e.g., a galactose recognition site) of the asialoglycoprotein receptor tends to be further improved, and the compound tends to be more efficiently introduced into hepatocytes.

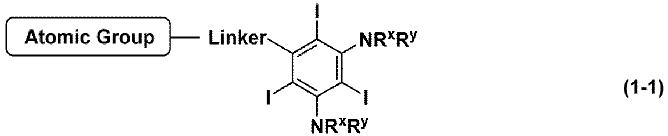

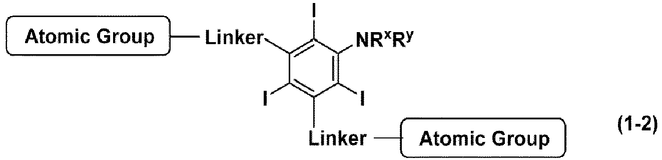

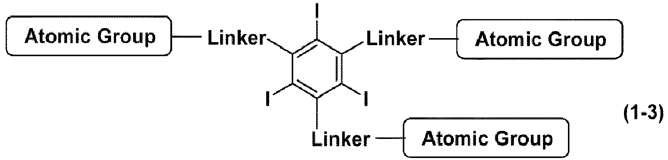

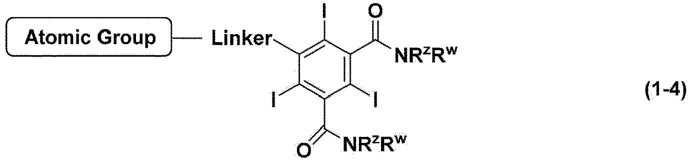

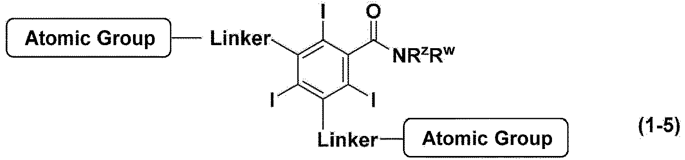

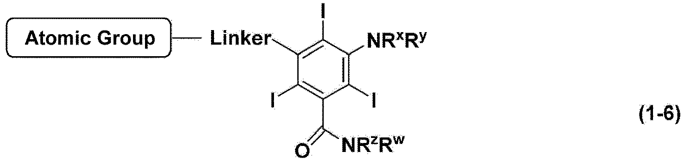

In the formulas (1-1) to (1-6), R^{x}, R^{y}, R^{z}, R^{w}, Atomic Group, and Linker have the same meanings with R^{x}, R^{y}, R^{z}, R^{w}, Atomic Group, and Linker in the formula (1) . When a plurality of -NR^{x}R^{y}, -C(=O)NR^{z}R^{w}, Atomic Groups, or Linkers are present, these moieties may be the same or different and are preferably the same.

In the formula (1), R^{x} and R^{y} in the amino group represented by -NR^{x}R^{y} each independently represent a hydrogen atom, a C1 to C6 hydrocarbon group optionally having a substituent or a C2 to C7 acyl group optionally having a substituent. R^{x} and R^{y} may be the same or different. Preferably, one of R^{x} and R^{y} represents a hydrogen atom, and the other moiety represents a C1 to C6 hydrocarbon group optionally having a substituent or a C2 to C7 acyl group optionally having a substituent. According to an alternative aspect, preferably, one of R^{x} and R^{y} represents a C1 to C6 hydrocarbon group optionally having a substituent, and the other moiety represents a C2 to C7 acyl group optionally having a substituent.

In R^{x} and R^{y}, the number of carbon atoms in the hydrocarbon group optionally having a substituent is preferably 1 or more and 5 or less, more preferably 1 or more and 4 or less. The number of carbon atoms in the acyl group optionally having a substituent is preferably 2 or more and 6 or less, more preferably 2 or more and 5 or less.

R^{z} and R^{w} in the amide group represented by - C(=O)NR^{z}R^{w} each independently represent a hydrogen atom or a C1 to C6 hydrocarbon group optionally having a substituent. R^{z} and R^{w} may be the same or different. Preferably, one of R^{x} and R^{y} represents a hydrogen atom, and the other moiety represents a C1 to C6 hydrocarbon group optionally having a substituent.

In R^{z} and R^{w}, the number of carbon atoms in the hydrocarbon group optionally having a substituent is preferably 1 or more and 5 or less, more preferably 1 or more and 4 or less.

The C1 to C6 hydrocarbon group may be a saturated or unsaturated linear or branched or cyclic hydrocarbon group. Examples thereof include an alkyl group having 1 or more and 6 or less carbon atoms, an alkenyl group having 2 or more and 6 or less carbon atoms, an alkynyl group having 2 or more and 6 or less carbon atoms, and an aryl group having 6 carbon atoms. The C1 to C6 hydrocarbon group is preferably a saturated linear or branched hydrocarbon group.

In the present specification, examples of the alkyl group having 1 or more and 6 or less carbon atoms specifically include a methyl group, an ethyl group, a n-propyl group, an isopropyl group, a n-butyl group, an isobutyl group, a t-butyl group, a pentyl group, a hexyl group, a cyclopropyl group, a cyclobutyl group, a cyclopentyl group, and a cyclohexyl group.

In the present specification, examples of the alkenyl group having 2 or more and 6 or less carbon atoms specifically include an allyl group, a methallyl group, a 1-buten-1-yl group, a 2-buten-1-yl group, a 3-buten-1-yl group, and a 1-buten-3-yl group.

In the present specification, examples of the alkynyl group having 2 or more and 6 or less carbon atoms specifically include a 2-propyn-1-yl group, a 2-butyn-1-yl group, and a 3-butyn-1-yl group.

In the present specification, examples of the aryl group having 6 carbon atoms specifically include a phenyl group.

The C2 to C7 acyl group is a group represented by R-CO-, and R is, for example, an alkyl group having 1 or more and 6 or less carbon atoms, an alkenyl group having 2 or more and 6 or less carbon atoms, an alkynyl group having 2 or more and 6 or less carbon atoms, or an aryl group having 6 carbon atoms. Examples of the C2 to C7 acyl group specifically include an acetyl group, an acryloyl group, and a benzoyl group.

Examples of the substituents for R^{x}, R^{y}, R^{z} and R^{w} include, but are not particularly limited to, a hydroxy group, a halogen atom, an organic oxy group represented by -OR^{A}, and an amino group represented by -N(R^{B})(R^{C}). These substituents can be contained in any moiety of the compound of the present invention, not limited to R^{x}, R^{y}, R^{z} and R^{w}.

Examples of the halogen atom can include a fluorine atom, a chlorine atom, a bromine atom, and an iodine atom.

Examples of R^{A} include an alkyl group having 1 or more and 6 or less carbon atoms, an alkenyl group having 2 or more and 6 or less carbon atoms, an alkynyl group having 2 or more and 6 or less carbon atoms, and an aryl group having 6 carbon atoms.

Examples of R^{B} and R^{C} each independently include a hydrogen atom, an alkyl group having 1 or more and 6 or less carbon atoms, an alkenyl group having 2 or more and 6 or less carbon atoms, an alkynyl group having 2 or more and 6 or less carbon atoms, and an aryl group having 6 carbon atoms.

The amino group represented by -NR^{x}R^{y} is preferably an acetylamino group (-NHCOR wherein R is a C1 to C6 hydrocarbon group optionally having a substituent; the C1 to C6 hydrocarbon group optionally having a substituent is as defined in R^{x} and R^{y}). According to an alternative aspect, the amino group represented by -NR^{x}R^{y} is preferably represented by any of the following structures:

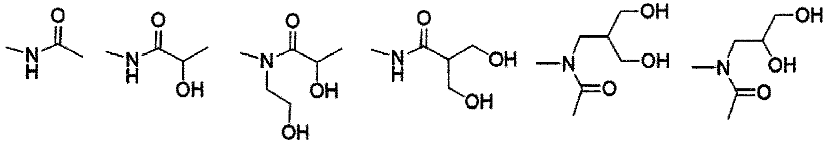

The amide group represented by -C(=O)NR^{z}R^{w} is preferably represented by any of the following structures:

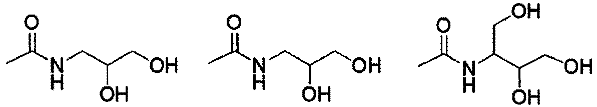

The left bond in the amino group represented by - NR^{x}R^{y} and the amide group represented by -C(=O)NR^{z}R^{w}, and a bond from NH or N, or a bond from C=O in each of the specifically shown structures each mean a bond to the triiodobenzene skeleton in the formula (1).

The Atomic Group in the compound of the present invention is an atomic group that binds to an asialoglycoprotein receptor, and is not particularly limited as long as the atomic group has a structure that is recognized by an asialoglycoprotein receptor. Examples of the structure that is recognized by an asialoglycoprotein receptor, of the Atomic Group include a sugar residue that binds to an asialoglycoprotein receptor, and a group of a peptide chain derived from immunoglobulin A (IgA) or the like. Examples of the Atomic Group include a group derived from a sugar residue that binds to an asialoglycoprotein receptor, and a group of a peptide chain derived from immunoglobulin A (IgA) or the like.

The Atomic Group in the compound of the present invention is preferably a sugar residue that binds to an asialoglycoprotein receptor, more preferably a group derived from galactose, N-acetylgalactosamine, N-trifluoroacetylgalactosamine, or galactose-N-acetylglucosamine (also referred to as "galactosyl-N-acetylglucosamine"). The galactose-N-acetylglucosamine means disaccharide in which galactose and N-acetylglucosamine are dehydratively condensed at the hydroxy group at position 1 of the galactose.

As used herein, the phrase "derived from galactose", "derived from N-acetylgalactosamine", "derived from N-trifluoroacetylgalactosamine", or "derived from galactose-N-acetylglucosamine" means that the galactose, the N-acetylgalactosamine, the N-trifluoroacetylgalactosamine, or the galactose-N-acetylglucosamine may be partially structurally modified for bonding to the Linker. Examples of the structural modification include the substitution of one hydroxy group in the galactose, the N-acetylgalactosamine, the N-trifluoroacetylgalactosamine, or the galactose-N-acetylglucosamine by a primary amino group (-NH₂).

The galactose, the N-acetylgalactosamine, the N-trifluoroacetylgalactosamine, or the galactose-N-acetylglucosamine may interact with the Linker via any functional group in this sugar. When the sugar residue that binds to an asialoglycoprotein receptor is bonded to the Linker, the carbon position to which the Linker is bonded can be position 1, 2, 3, 4, or 6 of the sugar and is preferably position 1.

The galactose-N-acetylglucosamine is not particularly limited as long as this sugar chain is recognized by an asialoglycoprotein receptor. The bond between galactose and N-acetylglucosamine can be a **β**1,3 bond or a **β**1,4 bond and is preferably a **β**1,3 bond.

The group represented by the formula (2) in the compound of the present invention is preferably a group represented by the formula (2-1) or the formula (2-2), more preferably a group represented by the formula (2-1). The group represented by the formula (2) may be a group represented by the formula (2-3) or (2-4).

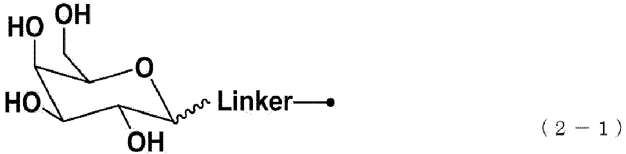

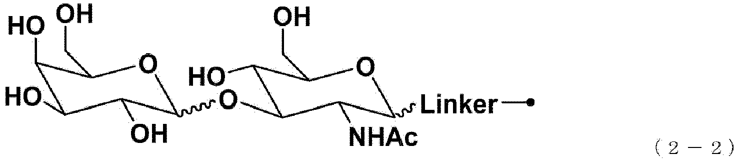

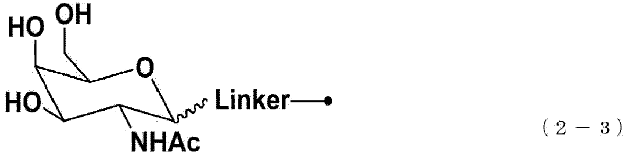

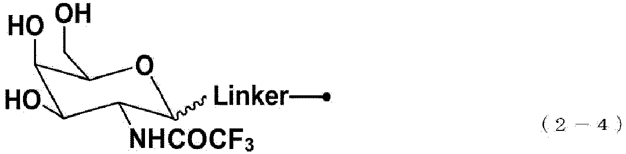

The Linker in each of the formulas (2-1), (2-2), (2-3) and (2-4) has the same meaning with the Linker in the formula (1). The bond between the group derived from galactose, N-acetylgalactosamine, N-trifluoroacetylgalactosamine, or N-acetylglucosamine and the Linker is represented by a wavy line, which means that the bond may be an **α** bond or a **β** bond. The bond of the wavy line is preferably a **β** bond.

The linker in the compound of the present invention refers to a structure that links the atomic group that binds to an asialoglycoprotein receptor to the triiodobenzene skeleton. When the compound of the present invention has two or more linkers, the two or more linkers may be the same or may be different and are preferably the same.

The linker in the compound of the present invention is preferably a hydrocarbon chain optionally having a substituent. In this aspect, one or both of the two ends of the hydrocarbon chain may have a heteroatom, an amide bond, an ester bond, a carbonyl bond, or an aromatic heterocycle. Specifically, the linker in the compound of the present invention can be a hydrocarbon chain optionally having a substituent which has a heteroatom, an amide bond, an ester bond, a carbonyl bond, and an aromatic heterocycle at one or both of its two ends. The hydrocarbon chain may contain an unsaturated bond and may be a linear or branched or cyclic hydrocarbon group. The hydrocarbon chain may be a combination of a linear and/or branched hydrocarbon and a cyclic hydrocarbon. Examples of the hydrocarbon chain include an alkylene chain, an alkenylene chain, an alkynylene chain, and an arylene group, and combinations thereof. The hydrocarbon chain may contain an atom other than a carbon atom and a hydrogen atom (e.g., an oxygen atom and a nitrogen atom) in its backbone. The hydrocarbon chain may have a carbonyl structure in the backbone.

The linker may have a heteroatom, an amide bond, an ester bond, a carbonyl bond, or an aromatic heterocycle at any of its two ends and preferably has a heteroatom, an amide bond, an ester bond, a carbonyl bond, or an aromatic heterocycle at both of its two ends. Specifically, in the compound of the present invention, each bond moiety between the atomic group that binds to an asialoglycoprotein receptor or the triiodobenzene skeleton and the linker is independently preferably a heteroatom, an amide bond, an ester bond, a carbonyl bond, or an aromatic heterocycle.

The hydrocarbon chain described above is more preferably an alkylene chain optionally having a substituent, or a hydrocarbon chain formed by bonding two or more alkylene chains optionally having a substituent via at least one selected from the group consisting of a heteroatom, an amide bond, an ester bond, a carbonyl bond, and an aromatic heterocycle. In this aspect, the alkylene chain may be linear or may have a branched chain. An element constituting the backbone of the alkylene chain described above may contain an atom other than a carbon atom, and the backbone of the alkylene chain described above is preferably constituted only by carbon atoms.

When an alkylene chain having the shortest bond between the atomic group that binds to an asialoglycoprotein receptor and the triiodobenzene skeleton is regarded as a backbone, the number of carbon atoms constituting the backbone is preferably 1 or more and 10 or less, more preferably 1 or more and 8 or less, further preferably 1 or more and 6 or less. The number of carbon atoms described above is more preferably 2 or more within the range described above and may be, for example, 2 or more and 6 or less.

When the number of carbon atoms falls within the range described above, the compound of the present invention easily interacts with a substrate recognition site (e.g., a galactose recognition site) of the asialoglycoprotein receptor and thus tends to be efficiently introduced into hepatocytes. Particularly, when the compound of the present invention has two or more (alternatively, three) groups represented by the formula (2) in the formula (1), the number of carbon atoms that falls within the range described above tends to further improve the interaction between the compound of the present invention and a substrate recognition site (e.g., a galactose recognition site) of the asialoglycoprotein receptor and tends to permit more efficient introduction into hepatocytes. In this aspect, the number of carbon atoms does not include the number of carbon atoms in functional groups, such as an aromatic heterocycle, an amide bond, an ester bond, and a carbonyl bond mentioned later, which may be contained in the alkylene chain.

Examples of the heteroatom include an oxygen atom, a sulfur atom, and a nitrogen atom. Such a heteroatom is preferably contained as an ether bond (-O-), a thioether bond (-S-), or an amine bond (-NH-). The heteroatom may be contained in the alkylene chain or may be located at the end of the alkylene chain, i.e., the bond position to the atomic group that binds to an asialoglycoprotein receptor, or the triiodobenzene skeleton.

The amide bond, the ester bond, and the carbonyl bond are represented by -NH-CO-, -CO-O-, and -CO-, respectively. The amide bond, the ester bond, and the carbonyl bond may each be contained in the alkylene chain or may each be located at the end of the alkylene chain, i.e., the bond position to the atomic group that binds to an asialoglycoprotein receptor, or the triiodobenzene skeleton.

Examples of the aromatic heterocycle include a triazole ring. Particularly, 1,2,3-triazole is preferred. The aromatic heterocycle may be contained in the alkylene chain or may be located at the end of the alkylene chain, i.e., the bond position to the atomic group that binds to an asialoglycoprotein receptor, or the triiodobenzene skeleton.

One or two or more of aromatic heterocycles, heteroatoms, amide bonds, ester bonds, and carbonyl bonds may be contained in one alkylene chain. The total number of aromatic heterocycles, heteroatoms, amide bonds, ester bonds, and carbonyl bonds contained in one hydrocarbon chain is preferably 2 or more, more preferably 2 or more and 6 or less, further preferably 2 or more and 4 or less.

The orientation of the amide bond or the ester bond is not particularly limited. The amide bond may be -NH-CO- or may be -CO-NH-. The ester bond may be -CO-O- or may be -O-CO-.

The bond position and orientation of the aromatic heterocycle are not particularly limited. For example, when the alkylene chain contains 1,2,3-triazole, this moiety may be bonded at, for example, 1-position and 4-position. When the 1,2,3-triazole is bonded at 1-position and 4-position, the 1-position may be located on the side of the atomic group that binds to an asialoglycoprotein receptor or the 4-position may be located on the side of the atomic group that binds to an asialoglycoprotein receptor.

In the linker in the compound of the present invention, when an alkylene chain having the shortest bond between the atomic group that binds to an asialoglycoprotein receptor and the triiodobenzene skeleton is regarded as a backbone, the number of carbon atoms constituting the backbone is preferably 1 or more and 15 or less, more preferably 1 or more and 13 or less, further preferably 1 or more and 12 or less. The number of carbon atoms constituting the backbone is preferably 2 or more, 3 or more, or 4 or more and may be 5 or more within the range described above. The number of carbon atoms may be 11 or less within the range described above. When the number of carbon atoms falls within the range described above, the compound of the present invention easily interacts with a substrate recognition site (e.g., a galactose recognition site) of the asialoglycoprotein receptor and thus tends to be efficiently introduced into hepatocytes. The number of carbon atoms described above may be in a range obtained by arbitrarily combining the upper limit value and the lower limit value described above.

Examples of the substituents that may be carried by the hydrocarbon chain and the alkylene chain described above include the same as the substituents for R^{x}, R^{y}, R^{z} and R^{w}. Each of the substituents is preferably selected from a hydroxy group and an amino group (-NH₂) and is more preferably a hydroxy group. Each of the substituents may be an alkyl group substituted by a hydroxy group or may be a side chain substituted by a hydroxy group and an alkyl group substituted by a hydroxy group.

Preferred examples of the form of the linker in the compound of the present invention include a linker represented by the following structure:

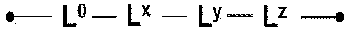

In the formula, L° is -O- or -NHC(=O)-, and L^{x} is represented by the following structure: 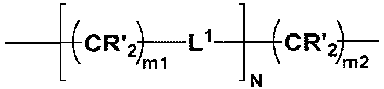 L^{y} represents a single bond or is represented by the following structure (preferably a single bond): 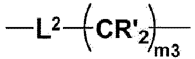 L^{z} is represented by the following structure:

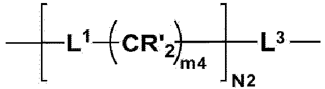

In the formulas, each R' is independently one selected from a hydrogen atom, a C1 to C3 alkyl group optionally having a substituent, and a hydroxy group; each L¹ is independently one selected from an ether bond (-O-), a thioether bond (-S-), an amine bond (-NH-), an amide bond, an ester bond, and a carbonyl bond; L² is an amide bond, or a divalent group derived from an aromatic heterocycle; and L³ is one selected from -OCH₂-, -NHCH₂-, -C(=O)NH-, -C(=O)NHCH₂-, and -NHC(=O)-.
m1, m2, and m4 are each independently an integer of 1 or larger; m3 is an integer of 0 or larger; and N and N2 are each independently an integer of 0 or larger.

L° represents the bond moiety between the linker and the atomic group that binds to an asialoglycoprotein receptor. L° is preferably -O- (ether bond). When L° is -NHC(=O)-, the atomic group that binds to an asialoglycoprotein receptor and the Linker are bonded in the following order: (atomic group that binds to an asialoglycoprotein receptor)-NHC(=O)-Linker.

In L^{x}, L^{y}, and L^{z}, examples of the C1 to C3 alkyl group optionally having a substituent, represented by R' include a methyl group, an ethyl group, a n-propyl group, and an isopropyl group. Examples of the substituent that may be carried by the C1 to C3 alkyl group described above include the same as the substituents for R^{x}, R^{y}, R^{z} and R^{w}. The substituent is preferably selected from a hydroxy group and an amino group (-NH₂).

In L¹, the orientation of the amide bond or the ester bond is not particularly limited. The amide bond may be -NH-CO- or may be -CO-NH-. The ester bond may be -CO-O- or may be -O-CO-. The same holds true for the amide bond represented by L².

In L², the divalent group derived from an aromatic heterocycle means a divalent group obtained by removing two hydrogen atoms from an aromatic heterocycle.

Examples of such a divalent group include, but are not particularly limited to, a divalent group obtained by removing hydrogen atoms at 1-position and 4-position from 1,2,3-triazole. In this case, the orientation of the divalent group derived from 1,2,3-triazole is not limited. The 1-position may be bonded to L^{x}, or the 4-position may be bonded to L^{x}.

Preferred examples of the form of L¹ include an ether bond and an amide bond. Preferred examples of the form of L³ include -C(=O)NHCH₂- and -NHC(=O)-.
m1, m2, and m4 are each independently an integer of 1 or larger, preferably an integer of 1 or larger and 5 or smaller, more preferably an integer of 1 or larger and 4 or smaller or 1 or larger and 3 or smaller, m1, m2, and m4 may each independently an integer of 2 or larger. m2 may be 1 or larger and 10 or smaller, 1 or larger and 8 or smaller, or 1 or larger and 6 or smaller.
m3 is an integer of 0 or larger and may be an integer of 1 or larger and 5 or smaller or may be an integer of 1 or larger and 4 or smaller or 1 or larger and 3 or smaller.
N and N2 are each independently an integer of 0 or larger. N and N2 may each independently be 5 or smaller and are each independently preferably 4 or smaller, more preferably 3 or smaller. The sum of N and N2 is preferably 0 or larger and 4 or smaller, more preferably 1 or larger and 3 or smaller.

In the linker described above, L^{x} is preferably any of the following structures: 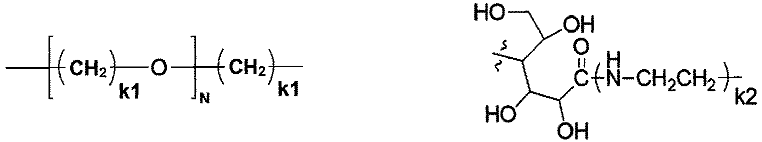 L^{y} is preferably a single bond or any of the following structures: 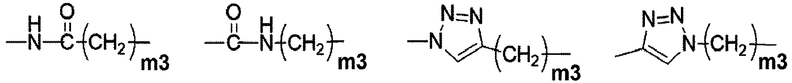 L^{z} is preferably any of the following structures:

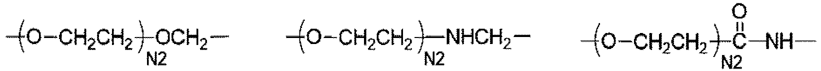

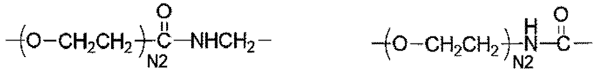

In the formulas, each k1 is independently an integer of 1 or larger and 3 or smaller; k2 is 0 or 1; m3 is an integer of 0 or larger; and N and N2 are each independently an integer of 0 or larger.

Each k1 is independently an integer of 1 or larger and 3 or smaller. When N is 2 or larger, a plurality of k2 can be the same or may be different.

Preferred numeric ranges of m3, N and N2 are the same as above.

Another preferred example of the form of the linker in the compound of the present invention includes a linker represented by the following structure:

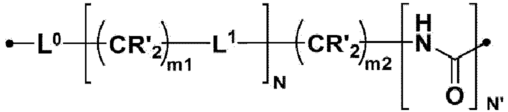

In the formula, each R' is independently one selected from a hydrogen atom, a C1 to C3 alkyl group optionally having a substituent, and a hydroxy group,
L° is -O- or -NHC(=O)-,
each L¹ independently represents a single bond or is one selected from an ether bond (-O-), a thioether bond (-S-), an amine bond (-NH-), an amide bond, an ester bond, and a carbonyl bond,
m1 and m2 are each independently an integer of 1 or larger,
N is an integer of 0 or larger, and
N' is 0 or 1.

Examples of the C1 to C3 alkyl group in the C1 to C3 alkyl group optionally having a substituent include a methyl group, an ethyl group, a n-propyl group, and an isopropyl group. Examples of the substituent for the C1 to C3 alkyl group optionally having a substituent can include the same substituents as those for R^{x}, R^{y}, R^{z} and R^{w}_{.}

L° is as defined above and is preferably -O-(ether bond).
m1 and m2 are each independently preferably an integer of 1 or larger and 5 or smaller, more preferably an integer of 1 or larger and 4 or smaller, further preferably an integer of 1 or larger and 3 or smaller.
m2 may be 1 or larger and 10 or smaller, 1 or larger and 8 or smaller, or 1 or larger and 6 or smaller.

N is not particularly limited as long as N is an integer of 0 or larger. N is preferably 0 or larger and 5 or smaller, more preferably 0 or larger and 4 or smaller, further preferably 1 or larger and 3 or smaller.

When N is 0, the linker structure described above is represented by the following formula:

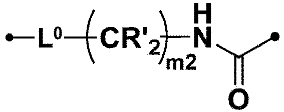

N' is 0 or 1. When N' is 0, the linker of the formula is represented by the formula given below. N' is preferably 1.

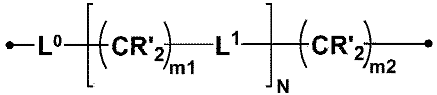

The linker in the compound of the present invention is further preferably represented by any of the following structures:

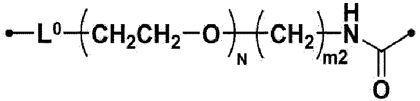

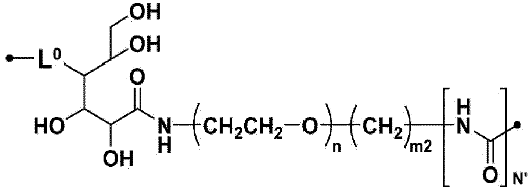

In the formulas, L° is -O- or -NHC(=O)-; N is an integer of 0 or larger; n is an integer of 0 or larger; m2 is an integer of 1 or larger; and N' is 0 or 1.

L° is as defined above and is preferably -O-(ether bond).
N is preferably 0 or larger and 5 or smaller, more preferably 0 or larger and 4 or smaller, further preferably 1 or larger and 3 or smaller.
n is preferably 0 or an integer of 1 or larger and 5 or smaller, more preferably 0 or an integer of 1 or larger and 4 or smaller, further preferably 0 or an integer of 1 or larger and 3 or smaller.
m2 can be an integer of 1 or larger and 10 or smaller, 1 or larger and 8 or smaller, or 1 or larger and 6 or smaller and is preferably an integer of 1 or larger and 5 or smaller, more preferably an integer of 1 or larger and 4 or smaller, further preferably an integer of 1 or larger and 3 or smaller.

When N or n is 0, m2 is preferably an integer of 1 or larger and 10 or smaller.

When N or n is an integer of 1 or larger, m2 is preferably an integer of 1 or larger and 6 or smaller.

N' is preferably 1.

The compound of the present invention may have an arbitrary combination of preferable forms respectively described about the amino group represented by -NR^{x}R^{y}, the amide group represented by -C(=O)NR^{z}R^{w}, and the group represented by the formula (2) in the formula (1). Likewise, preferable forms described about each group can be arbitrarily combined. Specifically, as one example, only the group represented by the formula (2), among the amino group represented by -NR^{x}R^{y}, the amide group represented by -C(=O)NR^{z}R^{w}, and the group represented by the formula (2), may be in a particular preferable form, or the amino group represented by -NR^{x}R^{y} and the amide group represented by -C(=O)NR^{z}R^{w}, among the amino group represented by -NR^{x}R^{y}, the amide group represented by - C(=O)NR^{z}R^{w}, and the group represented by the formula (2), may be in particular preferable forms respectively described thereabout, though the present invention is not particularly limited thereby.

A compound represented by any of the following formulas is preferable as the compound of the present invention:

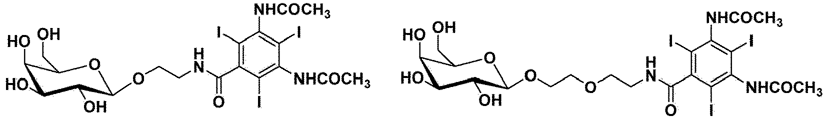

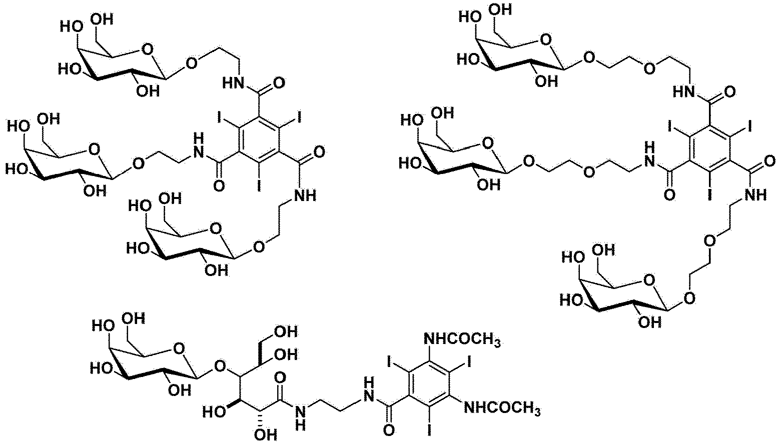

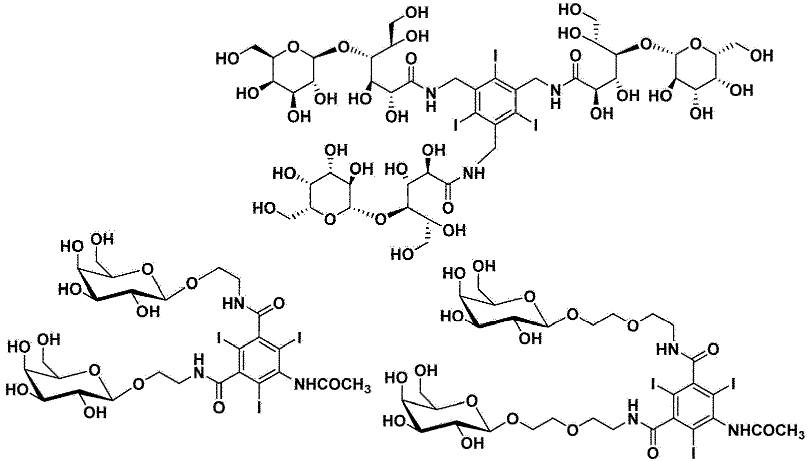

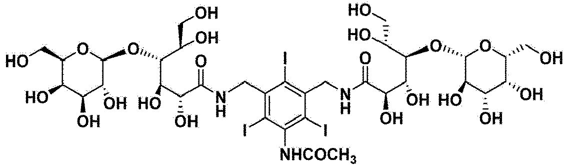

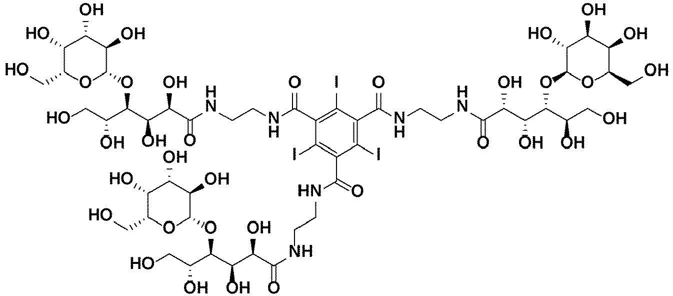

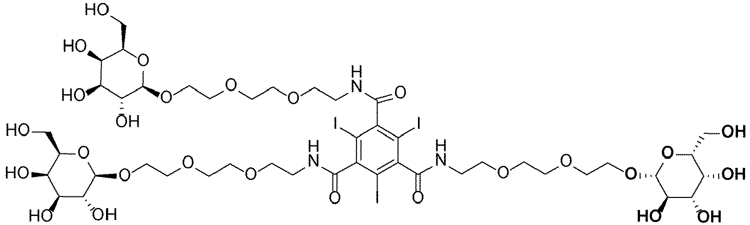

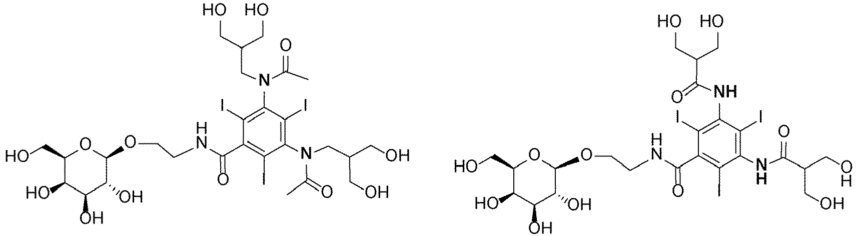

A compound represented by any of the following formulas is more preferable as the compound of the present invention:

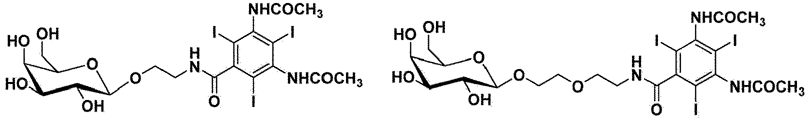

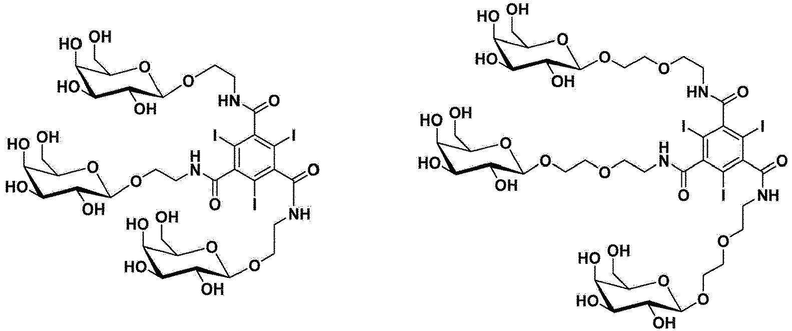

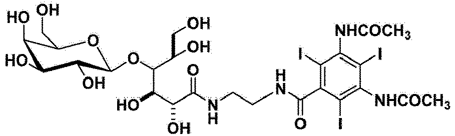

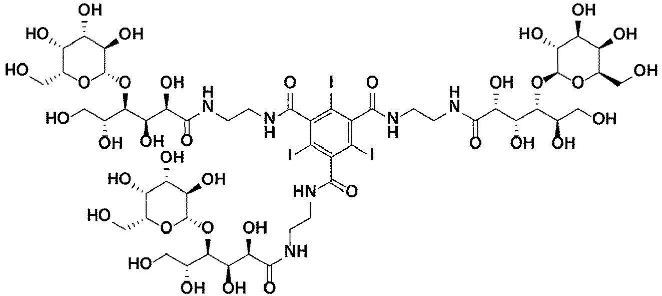

A compound represented by any of the following formulas is further preferable as the compound of the present invention:

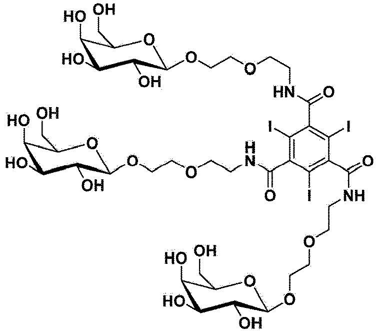

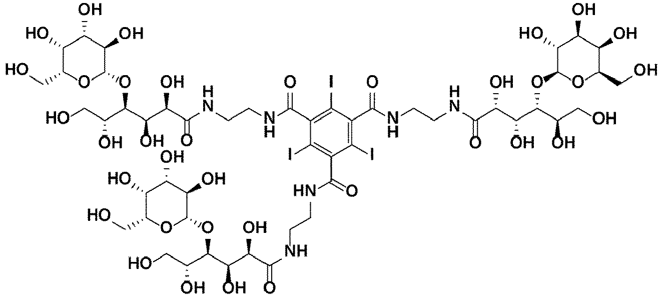

The respective structures of the linker moieties (i.e., structures that correspond to Linkers understood from the compounds represented by any of the formulas and the formulas (2-1) to (2-4)) in the compounds described above as preferable forms of the compound of the present invention serve as preferable Linker structures in the compound represented by the formula (1).

Specifically, an arbitrary linker as the Linker in the formula (1) can be Linker in the compound represented by any of the formulas, and an arbitrary linker as the Linker in each of the formulas (2-1) to (2-4) can be Linker in the compound represented by any of the formulas.

The linker described in the sentence "preferred examples of the form of the linker in the compound of the present invention include a linker represented by the following structure" can be Linker in the compound represented by any of the formulas.

Examples of the pharmaceutically acceptable salt of the compound of the present invention include inorganic acid salts such as hydrochloride, sulfate, and phosphate, and organic acid salts such as acetate, propionate, tartrate, fumarate, maleate, malate, citrate, methanesulfonate, p-toluenesulfonate, and trifluoroacetate.

Examples of the pharmaceutically acceptable salt of the compound of the present invention include alkali metal or alkaline earth metal salts such as sodium salt, potassium salt, and calcium salt.

### [Method for producing compound]

The compound of the present invention can be produced by use of an organic synthesis approach. The compound of the present invention is constituted by an atomic group moiety that binds to an asialoglycoprotein receptor, a linker moiety, and a nonionic iodine contrast agent moiety. Reaction substrates corresponding to the atomic group moiety that binds to an asialoglycoprotein receptor, the linker moiety, and the nonionic iodine contrast agent moiety can be reacted with each other for linking to produce the compound of the present invention.

The compound of the present invention can be produced by, for example, a method of reacting a reaction substrate constituted by an atomic group moiety that binds to an asialoglycoprotein receptor and a linker moiety with a reaction substrate of a nonionic iodine contrast agent moiety, or a method of introducing a linker moiety to a reaction substrate of a nonionic iodine contrast agent moiety, followed by reaction with a reaction substrate corresponding to an atomic group moiety that binds to an asialoglycoprotein receptor.

A method of reacting a reaction substrate constituted by an atomic group moiety that binds to an asialoglycoprotein receptor and a linker moiety with a reaction substrate of a nonionic iodine contrast agent moiety is preferable as a method for producing the compound of the present invention. The method for producing the compound represented by the formula (1) can be represented by, for example, the scheme given below. The present invention provides a method for producing the compound represented by the formula (1), comprising the step of reacting a reaction substrate constituted by an atomic group moiety that binds to an asialoglycoprotein receptor and a linker moiety with a reaction substrate of a nonionic iodine contrast agent moiety. The reaction substrate of a nonionic iodine contrast agent moiety is preferably a compound having a triiodobenzene skeleton.

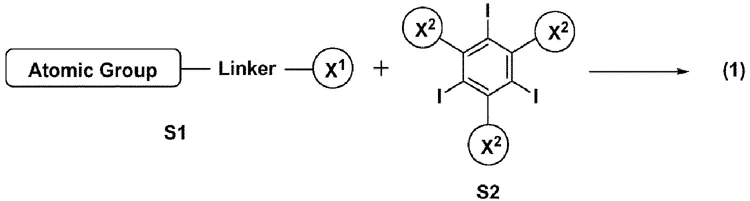

In the scheme, X¹ and at least one X² each contain a reactive group, and the reactive group of X¹ and the reactive group of X² react with each other to form a chemical bond. X² other than the reactive group is preferably -NR^{x}R^{y} or -C(=O)NR^{z}R^{w} wherein R^{x}, R^{y}, R^{z} and R^{w} are as defined above.

In this aspect, examples of the chemical bond to be formed include, but are not particularly limited to, a carbon-carbon bond (which may be a saturated bond or may be an unsaturated bond), an ether bond (-O-), a thioether bond (-S-), an amine bond (-NH-), an amide bond, an ester bond, a carbonyl bond and an aromatic heterocycle. Organic synthesis reaction known in the art can be applied to formation reaction for such a bond.

In the case of forming a carbon-carbon bond, examples of the reaction include, but are not particularly limited to, Wittig reaction, Grignard reaction, Suzuki-Miyaura coupling reaction, and Negishi coupling reaction. X¹ and X² can be organic groups that react through such reaction.

For the Wittig reaction, preferably, for example, one of X¹ and X² is an aldehyde group or a ketone group, and the other moiety is a phosphorus ylide group. For the Grignard reaction, preferably, for example, one of X¹ and X² is an aldehyde group or a ketone group, and the other moiety is a group (-MgX) constituting a Grignard reactant. For the Suzuki-Miyaura coupling reaction, preferably, for example, one of X¹ and X² is a boronic acid or boronic acid ester group, and the other moiety is a halogen group or a triflate group. For the Negishi coupling reaction, preferably, for example, one of X¹ and X² is a group (-ZnX) constituting organic zinc, and the other moiety is a halogen group.

In the case of forming an ether bond, preferably, for example, one of X¹ and X² is a hydroxy group, and the other moiety is preferably a halogen group.

In the case of forming a thioether bond, preferably, for example, one of X¹ and X² is a thiol group, and the other moiety is a halogen group.

In the case of forming an amine bond, preferably, for example, one of X¹ and X² is an amino group, and the other moiety is a halogen group.

In the case of forming an amide bond, preferably, for example, one of X¹ and X² is an amino group, and the other moiety is a carboxylic acid group, an acid halide group (-CO-X), or an acid anhydride group.

In the case of forming an ester bond, preferably, for example, one of X¹ and X² is a hydroxy group, and the other moiety is a carboxylic acid group or an acid halide group (-CO-X).

In the case of forming a carbonyl bond, preferably, for example, one of X¹ and X² is a Weinreb amide group (-CO-N(OMe)₂), and the other moiety is a group (-MgX) constituting a Grignard reactant or a group (-Li) constituting organic lithium.

In the case of forming an aromatic heterocycle, click reaction typified by Huisgen cyclization reaction can be used. For example, when one of X¹ and X² is an azide group (-N₃) and the other moiety is terminal alkyne, a 1,2,3-triazole ring can be formed.

Among the bonds to be formed, an amide bond is preferred. In this respect, preferably, X¹ is an amino group, and X² is a carboxylic acid group, an acid halide group (-CO-X), or an acid anhydride group. More preferably, X¹ is an amino group, and X² is an acid halide group (-CO-X).

Reaction substrate S1 constituted by an atomic group moiety that binds to an asialoglycoprotein receptor and a linker moiety (hereinafter, also referred to as intermediate S1), and reaction substrate S2 of a nonionic iodine contrast agent moiety (hereinafter, also referred to as intermediate S2) can be appropriately synthesized by organic synthesis approaches.

The intermediate S1 can be prepared, for example, by introducing a linker moiety to a group serving as Atomic Group, and subsequently introducing reactive group X¹ thereto. When the Atomic Group is a sugar residue structure as an example, the intermediate S1 is produced according to the scheme given below. Specifically, the intermediate S1 can be prepared by introducing a linker moiety to a sugar such as penta-O-acetyl-D-galactopyranose or hepta-O-acetyl-D-galactopyranosyl-N-acetylglucosamine or a derivative thereof as a starting material, and subsequently introducing reactive group X¹ thereto. The synthesis of the intermediate S1 may appropriately involve a conversion step necessary for the synthesis of the intermediate S1, such as the step of protection of a functional group with a protective group and/or deprotection.

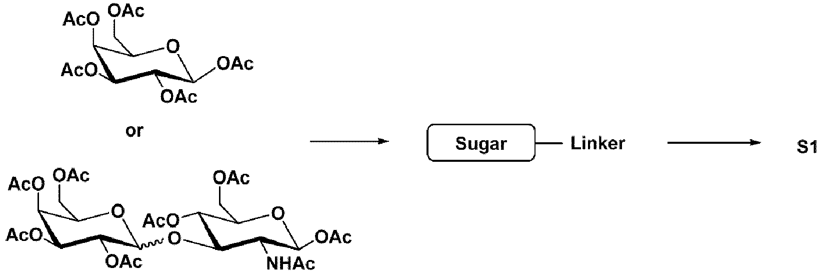

Before bonding between the atomic group moiety that binds to an asialoglycoprotein receptor and the linker moiety, a functional group of the atomic group moiety that binds to an asialoglycoprotein receptor may be converted to another substituent in advance. When the Atomic Group is a sugar residue structure as an example, a hydroxy group of the sugar moiety may be converted to another substituent in advance. Examples of such a substituent include a primary amino group and an acylamino group.

As described above, the functional group of the atomic group moiety that binds to an asialoglycoprotein receptor is converted to another substituent so that the atomic group moiety that binds to an asialoglycoprotein receptor can be bonded to the linker through an arbitrary bond. When the Atomic Group is a sugar residue structure as an example, a hydroxy group of the sugar moiety can be substituted by an amino group as described below. The resulting sugar moiety can be bonded to the linker through an amide bond.

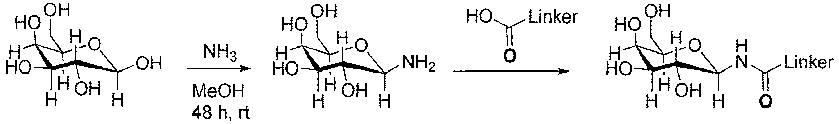

The introduction of the linker may be performed in stages. Specifically, the linker structure may be elongated through reaction of two or more stages and obtained as a final linker structure.

The intermediate S2 can be prepared, for example, as shown in the scheme given below, by introducing an iodo group to an aromatic compound having arbitrary substituents represented by R^{S1} to R^{S3} as a starting material, and subsequently introducing reactive group X² thereto to induce S2. The synthesis of the intermediate S2 may appropriately involve a conversion step necessary for the synthesis of the intermediate S2, such as the step of protection of a functional group with a protective group and/or deprotection.

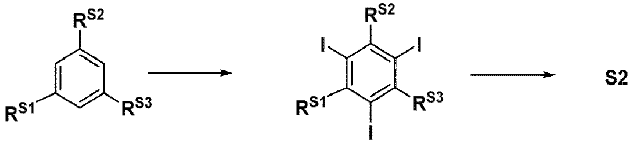

R^{S1} to R^{S3} preferably have different substituents depending on the number of atomic groups that bind to an asialoglycoprotein receptor to be introduced.

The compound of the present invention may be appropriately synthesized in the presence of a reaction solvent, a catalyst, an additive, or the like. Reaction conditions such as reaction temperature, reaction pressure, and reaction time can also be appropriately adjusted. The obtained product may be appropriately subjected to aftertreatment and then obtained as the compound of the present invention. Specific examples of the aftertreatment method can include extraction treatment and/or purification known in the art such as crystallization, recrystallization, and chromatography.

### [Contrast agent]

The compound of the present invention can be used as a contrast agent. Thus, one embodiment of the present invention is a contrast agent comprising the compound of the present invention.

An imaging technique to which the contrast agent of the present invention is applied is expected to be almost every contrast radiography that is performed using existing iodine contrast agents. Specifically, examples of the imaging technique include contrast CT examination from the head and neck to the chest, the upper and lower abdomens, and the extremities, and examination, treatment, etc. using vascular catheters in the brain, the heart, the aorta, the abdomen, and the like. This contrast agent is excreted into bile and urine and can therefore be expected to be applied to infusion urography, infusion cholangiography, and the like.

The compound of the present invention can be used in the form of a pharmaceutical composition known in the art as a contrast agent. The pharmaceutical composition can be produced by a conventional method known in the art.

One compound of the present invention may be used, or two or more compounds of the present invention may be used as a mixture.

The contrast agent of the present invention can contain a pharmaceutically acceptable additive. Examples of the additive include, but are not particularly limited to, stabilizers, excipients, binders, disintegrants, lubricants, antioxidants, corrigents, colorants, and flavors.

Although the contrast agent of the present invention is formulated so as to make it compatible with a therapeutically appropriate administration route including intravenous, intracutaneous, subcutaneous, oral (e.g., also including inhalation), percutaneous and transmucosal administration, it is suitably intravenously injected by utilizing the property of being excellent in handleability.

The dose of the contrast agent of the present invention is appropriately determined depending on the state of a recipient patient, an administration method, etc.

The present invention further provides a pharmaceutical composition comprising the compound represented by the formula (1) or a pharmaceutically acceptable salt thereof. The pharmaceutical composition may further comprise a pharmaceutically acceptable additive. The pharmaceutical composition may be used as a contrast agent.

The present invention also provides a contrast radiography method using the pharmaceutical composition comprising the compound represented by the formula (1) or a pharmaceutically acceptable salt thereof.

The present invention also provides the compound represented by the formula (1) or a pharmaceutically acceptable salt thereof for use in contrast radiography.

### Examples

Hereinafter, the present embodiment will be described in detail with reference to Examples. However, the present invention is not limited by the following Examples.

### [Example 1] Synthesis of MEG-1

For the synthesis of MEG-1, intermediate I and intermediate II were first synthesized.

### <Synthesis of intermediate I>

The intermediate I was synthesized according to the following scheme.

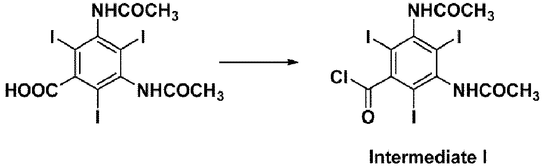

Commercially available diatrizoic acid (manufactured by Tokyo Chemical Industry Co., Ltd., 2.01 g, 3.27 mmol) was dissolved in thionyl chloride (20.0 mL), and the solution was refluxed at 120°C for 3 hours using an oil bath. Thionyl chloride was distilled off, and the residue was then washed with n-hexane to obtain intermediate I (1.39 g, 67%) as a yellowish-white powder.

¹H-NMR of the intermediate I was as follows. ¹H-NMR (500 MHz, DMSO-D₆) δ: 10.13 (s, 1H), 10.04 (s, 1H), 2.03 (s, 6H)

### <Synthesis of intermediate II>

The intermediate II was synthesized according to the following scheme.

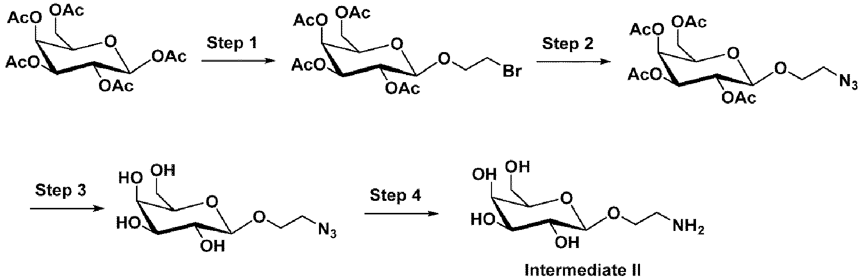

### (Step 1)

For reaction conditions of step 1 and subsequent step 2, Japanese Patent No. 4293735 was referred to.

Specifically, commercially available penta-O-acetyl-β-D-galactopyranose (manufactured by Tokyo Chemical Industry Co., Ltd., 3.89 g, 9.97 mmol) and 2-bromoethanol (0.71 mL, 9.45 mmol) were first dissolved in dehydrated methylene chloride (41.0 mL) under argon. Next, boronic acid (4.11 mL, 32.1 mmol) was gradually added dropwise to the solution under ice cooling, and the mixture was stirred for 1 hour and then stirred overnight in the dark. The reaction solution thus stirred was turned into orange color. The reaction was terminated by the addition of water to the reaction solution, followed by concentration. An appropriate amount of ethyl acetate was added to the reaction solution, which was then separated from the aqueous layer, washed with a saturated aqueous solution of sodium carbonate, and saturated brine. The organic layer was dried using sodium sulfide, then filtered, and concentrated. Then, the residue was purified by silica gel column chromatography (n-hexane: ethyl acetate = 3: 2) to obtain a bromide (3.03 g, 67%) as a clear colorless candy-like substance.

¹H-NMR of the bromide was as follows.

¹H-NMR (500 MHz,CDCl₃) δ: 5.40 (dd, J = 3.5, 1.0 Hz, 1H), 5.24 (dd, J = 10.9, 8.0 Hz, 1H), 5.03 (dd, J = 10.3, 3.4 Hz, 1H), 4.54 (d, J = 8.0 Hz, 1H), 4.17-4.21 (m, 2H), 4.12 (dd, J = 11.2, 6.6 Hz, 1H), 3.92 (td, J = 6.6, 1.1 Hz, 1H), 3.80-3.85 (m, 1H), 3.46-3.51 (m, 2H), 2.16 (s, 3H), 2.09 (s, 3H), 2.06 (s, 3H), 1.99 (s, 3H)

### (Step 2)

The bromide (3.02 g, 6.66 mmol) obtained in step 1 and sodium azide (1.00 g, 15.4 mmol) were dissolved in dehydrated dimethylformamide (35.0 mL) under argon, and the solution was stirred overnight at 80°C. An appropriate amount of ethyl acetate was added to the reaction solution, which was then separated from the aqueous layer, washed with a saturated aqueous solution of sodium carbonate, and saturated brine. The organic layer was dried using sodium sulfide, then filtered, and concentrated. Then, the residue was purified by silica gel column chromatography (n-hexane: ethyl acetate = 3: 2) to obtain an azide (2.37 g, 86%) as a clear colorless oily substance.

¹H-NMR of the azide was as follows.

¹H-NMR (500 MHz, CDCl₃) δ: 5.41 (dd, J = 3.5, 1.5 Hz 1H), 5.27 (dd, J = 10.5, 8.0 Hz 1H), 5.04 (dd, J = 10.0, 3.5 Hz 1H), 4.56 (d, J = 8.0 Hz 1H), 4.11-4.21 (m, 2H), 4.03-4.07 (m, 1H), 3.91 (td, J = 7.0, 1.0 Hz 1H), 3.68-3.72 (m, 1H), 3.49-3.54 (m, 1H), 3.29-3.33 (m, 1H), 2.16 (s, 3H), 2.07 (s, 3H), 2.06 (s, 3H), 1.99 (s, 3H)

### (Step 3)

For reaction conditions of step 3 and subsequent step 4, Journal of Medicinal Chemistry, 2005, 48, 645-652 was referred to.

Specifically, the azide (1.75 g, 4.21 mmol) obtained in step 2 was dissolved in dehydrated methanol (40.0 mL) under argon. A solution of sodium methoxide in methanol (1 M in MeOH) was added to the solution until pH reached 9. Then, the mixture was stirred at room temperature (TLC: iso-PrOH-water = 7:3, Rf = 0.810) until the reaction completed. The reaction solution was neutralized using an ion-exchange resin (12.0 g, Amberlite H+). The solution was filtered and then concentrated with an evaporator to obtain a deacetylated compound (1.02 g, 97%) as a yellow or brown oily substance.

¹H-NMR of the deacetylated compound was as follows. ¹H-NMR (500 MHz, D₂O) δ: 4.29 (d, J = 8.0 Hz, 1H), 3.77-3.78 (m, 1H), 3.64-3.71 (m, 1H), 3.53-3.62 (m, 4H), 3.50 (dd, J = 10.0, 3.7 Hz, 1H), 3.36-3.41 (m, 3H)

### (Step 4)

The deacetylated compound (52.9 mg, 0.212 mmol) obtained in step 3 and a palladium carbon catalyst (10% Pd-C, 6.70 mg) were added to dehydrated methanol (4.50 mL) for hydrogen substitution. The mixture was stirred at room temperature for 20 hours, then filtered, and concentrated using an evaporator to obtain intermediate II (43.3 mg, 92%) as a yellow or brown solid.

¹H-NMR (500 MHz, D₂O) δ: 4.26 (d, J = 7.4 Hz, 1H), 3.76-3.81 (m, 1H), 3.63 (d, J = 4.0 Hz, 1H), 3.53-3.61 (m, 4H), 3.50 (dd, J = 10.0, 3.7 Hz, 1H), 3.38 (dd, J = 9.7, 8.0 Hz, 1H), 2.70-2.73 (m, 2H)

### <Synthesis of MEG-1>

MEG-1 was synthesized according to the following scheme.

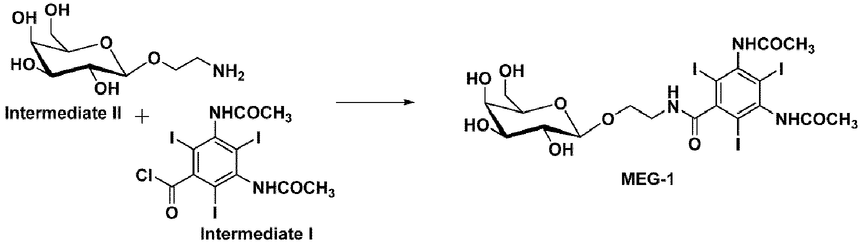

Specifically, the intermediate II (29.1 mg, 0.130 mmol) and the intermediate I (82.2 mg, 0.130 mmol) were first dissolved in dehydrated DMF (4.00 mL). Subsequently, triethylamine (19.9 µmL) was gradually added dropwise to the solution, and the mixture was stirred overnight at room temperature. The solvent was concentrated using an evaporator, and the residue was purified by silica gel column chromatography (chloroform: methanol = 4: 1) to obtain crude MEG-1 (38.6 mg, 36%) as a yellow or brown solid. Then, the solid was further purified by size-exclusion chromatography (SEC) to obtain MEG-1 as clear colorless crystals.

¹H-NMR of MEG-1 was as follows.

¹H-NMR (500 MHz, D₂O) δ: 4.31-4.35 (m, 1H), 3.80-3.99 (m, 1H), 3.78-3.88 (m, 1H), 3.74 (s, 1H), 3.46-3.66 (m, 6H), 3.36-3.40 (m, 1H), 2.11 (s, 6H)

### [Example 2] Synthesis of MEG-2

MEG-2 was synthesized according to the scheme given below in accordance with the method for synthesizing MEG-1 in Example 1. First, intermediate II' was synthesized.

### <Synthesis of intermediate II'>

The intermediate II' was synthesized according to the following scheme.

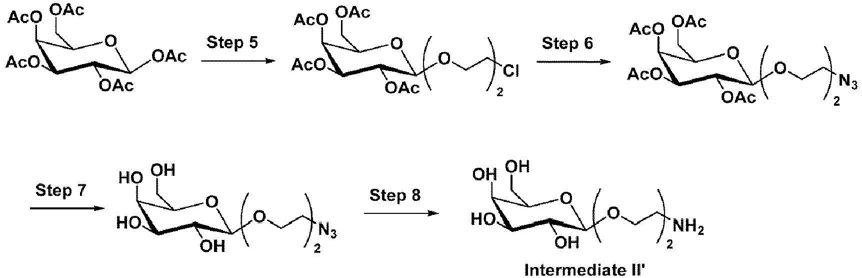

### (Step 5)

For reaction conditions of step 5 and subsequent step 6, Chinese Journal of Chemistry, 2006, 24, 1058-1061 was referred to.

Specifically, commercially available penta-O-acetyl-β-D-galactopyranose (manufactured by Tokyo Chemical Industry Co., Ltd., 3.91 g, 10.0 mmol) and 2-(2-chloroethoxy)ethanol (1.70 mL, 16.0 mmol) were first dissolved in dehydrated methylene chloride (41.0 mL) under argon. Subsequently, boronic acid (3.90 mL, 30.4 mmol) was gradually added dropwise to the solution under ice cooling, and the mixture was stirred for 1 hour and then stirred overnight in the dark. The reaction solution thus stirred was turned into orange color. The reaction was terminated by the addition of water to the reaction solution, followed by concentration. An appropriate amount of ethyl acetate was added to the reaction solution, which was then separated from the aqueous layer, washed with a saturated aqueous solution of sodium carbonate, and saturated brine. The organic layer was dried using sodium sulfide, then filtered, and concentrated. Then, the residue was purified by silica gel column chromatography (n-hexane: ethyl acetate = 3: 2) to obtain a chloride as a clear colorless oily substance.

¹H-NMR of the chloride was as follows.

¹H-NMR (500 MHz, CDCl₃) δ: 5.39 (s, 1H), 5.19-5.23 (m, 1H), 5.02 (td, J = 6.7, 3.4 Hz, 1H), 4.59 (dd, J = 7.7, 2.0 Hz, 1H), 4.09-4.20 (m, 2H), 3.91-3.99 (m, 2H), 3.72-3.78 (m, 3H), 3.61-3.69 (m, 4H), 2.15 (s, 3H), 2.07 (s, 3H), 2.05 (s, 3H), 1.99 (s, 3H)

### (Step 6)

The chloride (1.84 g, 4.06 mmol) obtained in step 5 and sodium azide (0.612 g, 9.41 mmol) were dissolved in dehydrated dimethylformamide (35.0 mL) under argon, and the solution was stirred overnight at 80°C. An appropriate amount of ethyl acetate was added to the reaction solution, which was then separated from the aqueous layer, washed with a saturated aqueous solution of sodium carbonate, and saturated brine. The organic layer was dried using sodium sulfide, then filtered, and concentrated. Then, the residue was purified by silica gel column chromatography (n-hexane: ethyl acetate = 3: 2) to obtain an azide (1.75 g, 94%) as a transparent yellow oily substance.

¹H-NMR of the azide was as follows.

¹H-NMR (500 MHz, CDCl₃) δ: 5.39 (d, J = 3.4 Hz, 1H), 5.22 (dd, J = 10.9, 8.0 Hz, 1H), 5.01 (dd, J = 11.0, 3.5 Hz 1H), 4.59 (d, J = 8.0 Hz, 1H), 4.11-4.20 (m, 2H), 3.91-3.99 (m, 2H), 3.75-3.79 (m, 1H), 3.62-3.71 (m, 4H), 3.38 (m, 2H), 2.15 (s, 3H), 2.08 (s, 3H), 2.05 (s, 3H), 1.99

### (s, 3H)

### (Step 7)

For reaction conditions of step 7, Org. Biomol. Chem., 2018, 16, 8413-8419 was referred to.

Specifically, the azide (383 mg, 0.830 mmol) obtained in step 6 was dissolved in dehydrated methanol (18.0 mL) under argon. A solution of sodium methoxide in methanol (1 M in MeOH) was added to the solution until pH reached 9. Then, the mixture was stirred at room temperature (TLC: iso-PrOH-water = 7:3, Rf = 0.818) until the reaction completed. The reaction solution was neutralized using an ion-exchange resin (3.00 g, Amberlite H+). The solution was filtered and then concentrated with an evaporator to obtain a deacetylated compound (1.02 g, 97%) as a brown oily substance.

¹H-NMR of the deacetylated compound was as follows. ¹H-NMR (500 MHz, CD₃OD) δ: 4.26-4.28 (d, J = 7.4 Hz, 1H), 3.99-4.03 (m, 1H), 3.82 (s, 1H), 3.65-3.77 (m, 9H), 3.45-3.54 (m, 3H)

### (Step 8)

For reaction conditions of step 8, J. Med. Chem., 2005, 48, 645-652 was referred to.

Specifically, the deacetylated compound (1.0 g, 3.64 mmol) obtained in step 7 and a palladium carbon catalyst (10% Pd-C, 0.135 g) were added to dehydrated methanol (35.0 mL) for hydrogen substitution. The mixture was stirred at room temperature for 18 hours, then filtered, and concentrated using an evaporator to obtain intermediate II' (833 mg, 86%) as a brown oily substance.

¹H-NMR of the intermediate II' was as follows. ¹H-NMR (500 MHz, CD₃OD) δ: 4.26-4.27 (d, J = 7.4 Hz, 1H), 3.97-4.04 (m, 1H), 381-3.82 (m, 1H), 3.66-3.77 (m, 9H), 3.52-3.56 (m, 3H)

### <Synthesis of MEG-2>

MEG-2 was synthesized according to the following scheme.

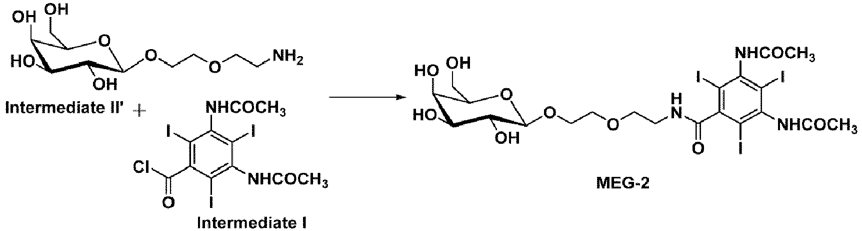

Specifically, the intermediate II' (67.0 mg, 0.251 mmol) and the intermediate I (159 mg, 0.252 mmol) were dissolved in dehydrated DMF (3.00 mL). Subsequently, triethylamine (34.8 µmL) was gradually added dropwise to the solution, and the mixture was stirred overnight at room temperature. The solvent was concentrated using an evaporator, and the residue was purified by silica gel column chromatography (chloroform: methanol = 4: 1) to obtain crude MEG-2 (31.0 mg, 16%) as a yellow or brown solid. Then, the solid was further purified by size-exclusion chromatography (SEC) to obtain MEG-2 as clear colorless crystals.

¹H-NMR of MEG-2 was as follows.

¹H-NMR (500 MHz, D₂O) δ: 4.24-4.26 (m, 1H), 3.89-3.93 (m, 1H), 3.74 (s, 1H), 3.42-3.69 (m, 11H), 3.32-3.37 (m, 1H), 2.10 (s, 6H)

### [Example 3] Synthesis of RYO-1

For the synthesis of RYO-1, intermediate III was first synthesized.

### <Synthesis of intermediate III>

The intermediate III was synthesized according to the scheme given below. The synthesis of the intermediate III was performed with reference to Jinyong Fan et al., Journal of Materials Science: Materials in Medicine, 2010, 21, 319-327.

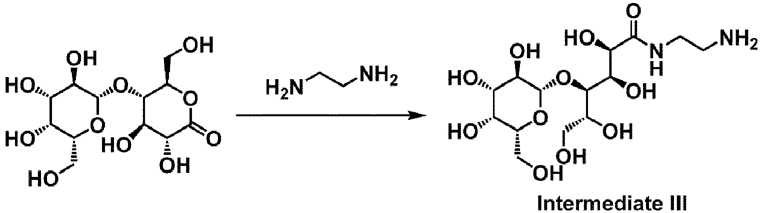

Specifically, a solution of starting materials lactone and excessive ethylenediamine in anhydrous dimethyl sulfoxide (DMSO) was first refluxed for 2 hours. The product intermediate III was precipitated by the addition of acetone, and the obtained yellow precipitates were dried under reduced pressure. Results of mass spectrometry of the obtained intermediate III were as follows.

LCMS (ESI) m/z [M+H]⁺ calcd for C₁₄H₂₉N₂O₁₁: 401;found: 401.

The lactone was synthesized according to the following scheme with reference to Alexandra. M. B et al., European polymer journal, 2012, 48, 963-973.

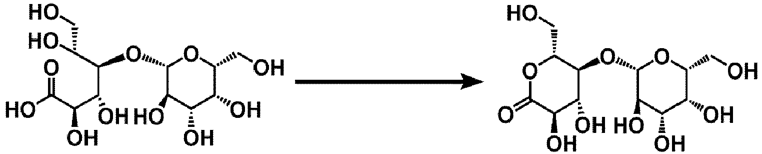

Specifically, an aqueous solution of desalted water (375 mL) containing 18.0 g (50 mmol) of 4-O-β-galactopyranosyl-D-gluconic acid (manufactured by FUJIFILM Wako Pure Chemical Corp.) was first heated at 40°C for 1 hour. Then, water was removed under reduced pressure, and the residue was washed with methanol (375 mL). Subsequently, methanol was evaporated, and this operation was repeated four times. This operation was further performed twice using 2-propanol. The lactone of interest was obtained as a white solid (14.19 g, 83%).

### <Synthesis of RYO-1>

RYO-1 was synthesized according to the following scheme in accordance with the method for synthesizing MEG-1 in Example 1.

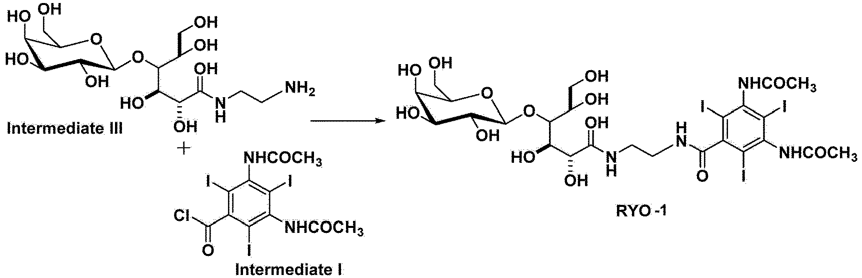

The intermediate III (100 mg, 0.25 mmol) was added at room temperature to a solution of the intermediate I dissolved in DMF (3 ml). Next, the reaction mixture was stirred overnight at 50°C. Then, the reaction mixture was directly separated by silica gel column chromatography (chloroform: methanol: water = 30: 20: 4; v/v) to obtain a white solid (Rf = 0.24; chloroform: methanol: water = 30: 20: 4; v/v). Subsequently, the product was separated by size-exclusion recycle HPLC (eluent: water, flow rate: 3 ml/mins), and a fraction containing the product was dried at 60°C to obtain RYO-1 as a white solid.

¹H-NMR of RYO-1 was as follows.

¹H-NMR (500 MHz, D₂O) δ: 8.04 (s, 1H), 4.38 (d, J = 7.4 Hz, 1H), 4.25 (d, J = 1.7 Hz, 1H), 4.05 (s, 1H), 3.33-3.83 (m, 14H), 2.08 (d, J = 2.3 Hz, 6H)

### [Example 4] Synthesis of MEG-3

For the synthesis of MEG-3, intermediate IV was first synthesized.

### <Synthesis of intermediate IV>

The intermediate IV was synthesized according to the following scheme.

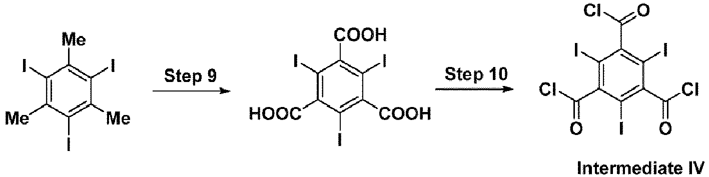

### (Step 9)

For reaction conditions of step 9, Chinese Patent No. 102503814 was referred to.

Specifically, a solution of triiodomesitylene (1.0 g, 2.0 mmol) in pyridine (30 ml) and water (10 ml) was stirred at 60°C for 10 minutes. The triiodomesitylene was synthesized with reference to U.S. Patent No. 6310243. Next, potassium permanganate (12 g, 75.9 mmol) was added thereto in small portions over 5 hours at 90°C. The reaction mixture was stirred for 24 hours, then filtered warm, and washed with a 5% aqueous potassium hydroxide solution. The filtrate was concentrated under reduced pressure at 60°C. Water was added to the residue, and the mixture was filtered to remove an insoluble white solid. pH was adjusted to 1 by the addition of a 5 N aqueous hydrochloric acid solution, and the filtrate was subjected to extraction with ethyl acetate three times. The organic phase was distilled off to obtain carboxylic acid as a white solid (0.82 g, 70%).

### (Step 10)

For reaction conditions of step 10, U.S. Patent No. 6310243 was referred to.

Specifically, the carboxylic acid (200 mg, 0.339 mmol) obtained in step 9 was placed in an eggplant flask, to which thionyl chloride (1 mL), and DMF (one drop) were then added, and the mixture was refluxed at 120°C for 2.5 hours. The solvent was distilled off under reduced pressure at 50°C. Then, the residue was dissolved in toluene (10 mL), and the solution was stirred at 50°C for 2 hours. Then, a solid was removed by filtration, and the filtrate was then concentrated and washed with a small amount of hexane to obtain intermediate IV (92.6 mg, 43%) as a white powder.

### <Synthesis of MEG-3>

MEG-3 was synthesized according to the following scheme.

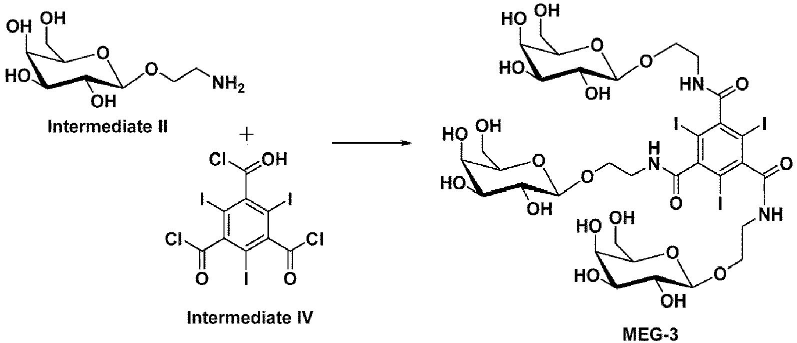

Specifically, the intermediate II (56.3 mg, 0.252 mmol) and the intermediate IV (50.1 mg, 0.0781 mmol) were first dissolved in dehydrated DMF (5.00 mL). Subsequently, triethylamine (30.0 µmL) was gradually added dropwise to the solution, and the mixture was stirred overnight at room temperature. The solvent was concentrated using an evaporator, and the residue was then purified three times by size-exclusion chromatography (SEC) to obtain MEG-3 as transparent brown crystals.

¹H-NMR of MEG-3 was as follows.

¹H-NMR (500 MHz, D₂O) δ: 4.36-4.36 (m, 1H), 3.96-4.01 (m, 1H), 3.81-3.86 (m, 1H), 3.76-3.79 (m, 1H), 3.50-3.60 (m, 6H), 3.39-3.42 (m, 1H)

### [Example 5] Synthesis of MEG-4

MEG-4 was synthesized according to the following scheme in accordance with the method for synthesizing MEG-3 in Example 4.

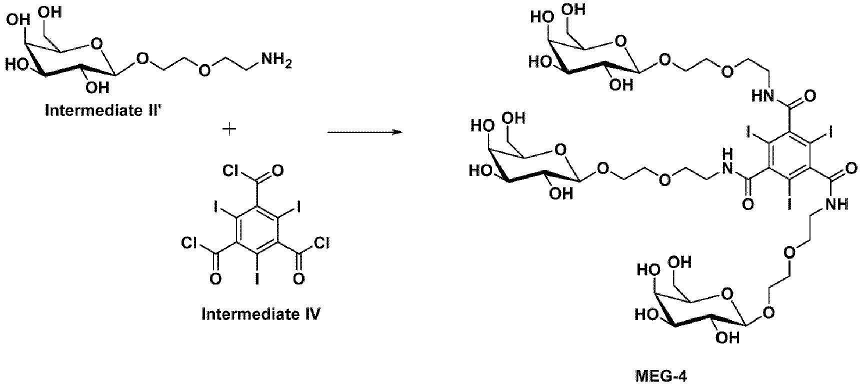

Specifically, the intermediate II' (25.1 mg, 0.0935 mmol) and the intermediate IV (20.0 mg, 0.0312 mmol) were dissolved in dehydrated DMF (3.30 mL). Subsequently, triethylamine (13.0 µmL) was gradually added dropwise to the solution, and the mixture was stirred overnight at room temperature. The solvent was concentrated using an evaporator, and the residue was then purified by size-exclusion chromatography (SEC) to obtain MEG-4 as a mixture.

¹H-NMR and results of mass spectrometry of MEG-4 were as follows.

¹H-NMR (500 MHz, D₂O) δ: 4.24-4.33 (dd, J = 7.4, 5.2 Hz, 1H), 3.87-3.94 (m, 3H), 3.79-3.80 (m, 3H), 3.51-3.71 (m, 33H), 3.34-3.46 (m, 3H); HRMS (ESI) m/z [M+Na]⁺ calcd for C₆₃H₈₄I₃N₃O₃₆: 1358.0592; found 1358.0599.

### [Example 6] Synthesis of KBT

For the synthesis of KBT, intermediate III' was first synthesized.

### <Synthesis of intermediate III'>

The intermediate III' was synthesized according to the following scheme.

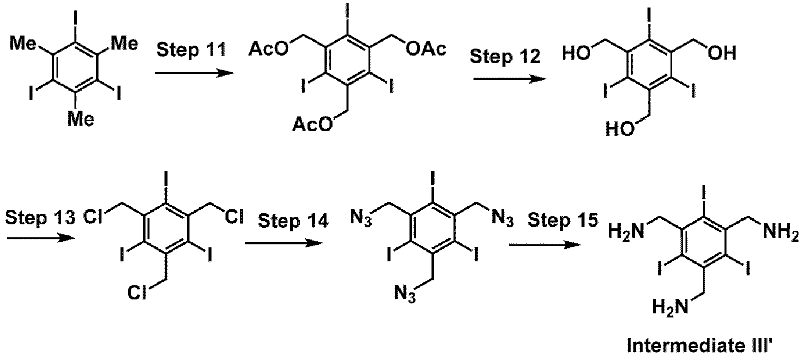

### (Step 11)

Triiodomesitylene (19.5 g, 39 mmol) was added to a mixture of glacial acetic acid (200 ml), acetic anhydride (400 ml), and concentrated sulfuric acid (40 ml). Potassium permanganate (24.6 g, 156 mmol) was added thereto in small portions over 3 hours. The mixture was stirred for 16 hours. Then, the solvent was distilled off, and water (200 ml) was added to the residue. The suspension was subjected to extraction with dichloromethane (250 ml). The organic phase was washed with water, dried over magnesium sulfate, and then distilled off. The solid residue was purified by silica gel chromatography (eluent: chloroform). 8.2 g of an acetylated compound (1,3,5-triiodo-2,4,6-triacetoxymethylbenzene) was obtained (yield: 31%).

¹H-NMR of the acetylated compound was as follows. ¹H-NMR (CDCl₃) δ: 5.70 (s, 1H), 2.11 (s, 2H)

### (Step 12)

The acetylated compound (7.56 g, 11.25 mmol) obtained in step 11 was suspended in methanol (120 ml), and K₂CO₃ (0.26 g, 1.9 mmol) was added to the suspension. The mixture was stirred at ambient temperature for 16 hours. Then, the reaction mixture was neutralized with a 2 M aqueous hydrochloric acid solution, and the organic solvent was then distilled off. The residue was suspended in water, and a white solid was collected by filtration and washed with water, methanol, and ether in order to obtain 6.0 g of an alcohol (1,3,5-triiodo-2,4,6-trihydroxymethylbenzene) (yield: 94%).

¹H-NMR of the alcohol was as follows.

¹H-NMR (DMSO-d₆) δ: 5.11-5.07 (s, 6H), 3.32 (s, 3H)

### (Step 13)

The alcohol (1.0 g, 1.83 mmol) obtained in step 12 was suspended in thionyl chloride (50 mL). Three drops of DMF were added to the suspension, and the mixture was heated to reflux for 3 hours. Thionyl chloride was removed under reduced pressure. The solid residue was suspended in toluene (20 ml), and the solution was poured to ice. The product was extracted with toluene, and the organic phase was washed with water three times and with saturated brine once. The organic phase was dried over sodium sulfate, then filtered, and concentrated to obtain 5.9 g of a chloride (1,3,5-tri(chloromethyl)-2,4,6-triiodobenzene) (yield: 97%).

¹H-NMR of the chloride was as follows.

¹H-NMR (CDCl₃) δ: 5.29 (s, 6H)

### (Step 14)

Sodium azide (65 mg, 1 mmol) was added to a solution of the chloride (0.1 g, 0.17 mmol) obtained in step 13 in DMSO in an argon atmosphere. The solution was stirred at 60°C for 1 hour, and the reaction was then terminated with water. The product was extracted with ethyl acetate three times, and the organic phase was washed with saturated brine. The organic phase was dried over anhydrous sodium sulfate, then filtered, and concentrated under reduced pressure to obtain azide (1,3,5-tri(azidomethyl)-2,4,6-triiodobenzene) (0.1017 g, yield: 98%) as a white solid.

¹H-NMR of the azide was as follows.

¹H-NMR (CDCl₃) δ: 5.20 (s, 6H) .

### (Step 15)

Triphenylphosphine (0.79 g, 3 mmol) was added to a solution of the azide (0.53 g, 0.85 mmol) obtained in step 14 in THF (5 ml) at 0°C in an argon atmosphere. The reaction mixture was stirred for 10 minutes. Water (0.8 ml) was added to this solution at 0°C, and the mixture was then stirred overnight at ambient temperature. The product was extracted with a 2 N aqueous hydrochloric acid solution, and the aqueous solution was washed with ether once to remove triphenylphosphine and washed with ethyl acetate four times to remove triphenylphosphine oxide. A 5 N aqueous potassium hydroxide solution was added to the aqueous phase until pH became approximately 14. Then, the product was extracted with chloroform three times. The organic layer was concentrated under reduced pressure to obtain intermediate III' as a white solid.

¹H-NMR of the intermediate III' was as follows. ¹H-NMR (CDCl₃) δ: 4.47 (s, 6H)

### <Synthesis of KBT>

KBT was synthesized according to the following scheme.

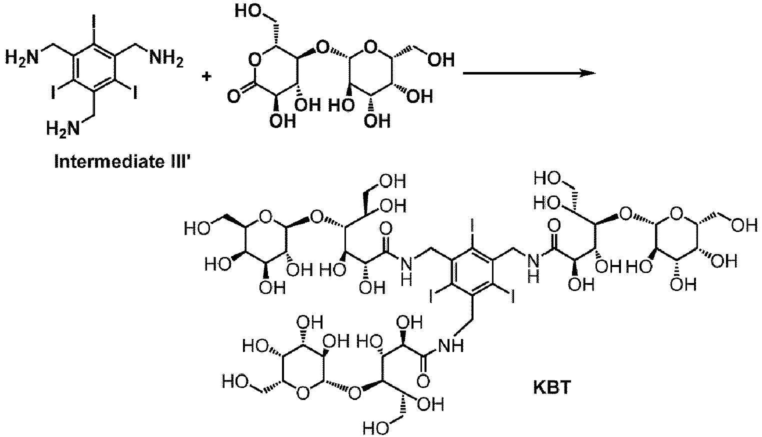

A mixture of the intermediate III' (10 mg, 0.018 mmol) and the lactone (27 mg, 0.079 mmol) used as a starting material in <Synthesis of intermediate III> in acetonitrile (3 mL) was refluxed for 120 hours. After cooling, chloroform and water were added to the mixture, followed by extraction with chloroform. The organic layer was concentrated under reduced pressure to obtain white crystals. The crystals were purified by size-exclusion chromatography using water as an eluent to obtain KBT.

¹H-NMR of KBT was as follows.

¹H-NMR (500 MHz, D₂O) δ: 4.85 (s, 6H), 4.40 (d, J = 7.4 Hz, 3H), 4.23-4.28 (m, 3H), 4.03 (br s, 3H), 3.86-3.39 (m, 33H)

### [Example 7] Synthesis of RYO-2

RYO-2 was synthesized according to the following scheme.

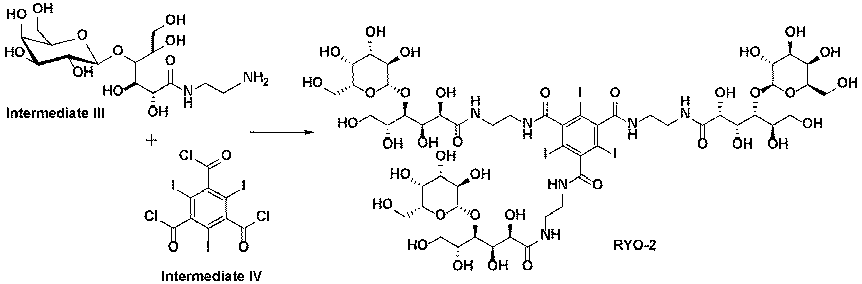

Specifically, the intermediate III (109 mg, 0.27 mmol) synthesized in the same manner as in Example 3 and DIPEA (diisopropylethylamine) (48 µL, 0.27 mmol) were added to a solution of the intermediate IV (50 mg, 0.78 mmol) dissolved in DMF (3 ml) at room temperature. Next, the reaction mixture was stirred overnight at 50°C. Then, the reaction mixture was directly separated by silica gel column chromatography (methanol: water = 1 : 1; v/v)) to obtain a white solid. Subsequently, the product was separated by size-exclusion recycle HPLC (eluent: water, flow rate: 5 ml/mins), and a fraction containing the product was dried at 60°C to obtain RYO-2 as a white solid.

¹H-NMR of RYO-2 was as follows.
¹H-NMR (500 MHz, D₂O) δ: 8.04 (s, 3H), 4.38 (d, J = 7.4 Hz, 3H), 4.25 (s, 3H), 4.05 (s, 3H), 3.81 (dd, J = 6.6, 4.3 Hz, 3H), 3.67-3.75 (m, 9H), 3.32-3.62 (m, 33H)

### [Example 8] Synthesis of PK-1 and PK-2

For the synthesis of PK-1, intermediate V' was first synthesized. Also, intermediate V for the synthesis of PK-2 can be synthesized as follows.

### <Synthesis of intermediates V' and V>

The intermediate V' was synthesized according to the scheme given below. The intermediate V can be synthesized according to the following scheme.

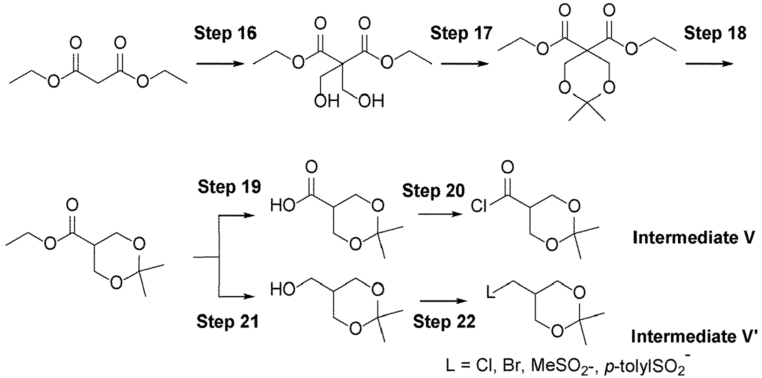

### (Step 16)

Diethyl malonate (15 mL, 99.4 mmol) was gradually added dropwise to a mixed solution of sodium bicarbonate (742 mg, 8.83 mmol) and a 37% aqueous formaldehyde solution (27 mL) at room temperature, and the mixture was then stirred for 4 hours. The reaction was terminated by the addition of saturated brine, and the product was extracted with diethyl ether four times. The combined organic layer was dried over sodium sulfate and then filtered, and the solvent was distilled off under reduced pressure. Diethyl 2,2-bis(hydroxymethyl)malonate (21,3 g, 98%) was obtained as a colorless oil (Rf = 0.31, ethyl acetate: n-hexane = 1: 1; v/v).

¹H-NMR of diethyl 2,2-bis(hydroxymethyl)malonate was as follows.

¹H-NMR (500 MHz, CDCl₃) δ: 4.15-4.26 (m, 4H), 4.06 (m, J = 12.0 Hz, 4H), 3.13-3.41 (m, 2H), 1.18-1.24 (m, 6H)

### (Step 17)

Diethyl 2,2-bis(hydroxymethyl)malonate (5.00 g, 22.9 mmol) and super dehydrated acetone (15 mL) were mixed in an argon atmosphere. Acetone dimethyl acetal (3.62 mL, 29.5 mmol) was added thereto, and subsequently, concentrated sulfuric acid (0.1 mL) was added thereto. The mixed reaction solution was stirred at room temperature for 24 hours. Then, a saturated aqueous solution of sodium bicarbonate was added thereto, and the mixture was stirred for 15 minutes. This mixed solution was filtered, and the filtrate was subjected to extraction with acetone twice. The solvent was distilled off under reduced pressure. A saturated aqueous solution of sodium bicarbonate was added to the residue, and the product was extracted with diethyl ether and washed with saturated brine. This extract was dried over sodium sulfate, filtered, and concentrated, and the residue was purified by neutral silica gel column chromatography to obtain diethyl 2,2-dimethyl-1,3-dioxane-5,5-dicarboxylate (4.25 g, 71%) as a colorless oil (Rf = 0.9, ethyl acetate: n-hexane = 1: 1; v/v).

¹H-NMR of diethyl 2,2-dimethyl-1,3-dioxane-5,5-dicarboxylate was as follows.

¹H-NMR (500 MHz, CDCl₃) δ: 4.25 (s, 4H), 4.20 (q, J = 7.1 Hz, 4H), 1.38 (s, 6H), 1.23 (t, J = 7.2 Hz, 6H)

### (Step 18)

A mixed solution of diethyl 2,2-dimethyl-1,3-dioxane-5,5-dicarboxylate (391 mg, 1.5 mmol), sodium chloride (106 mg, 1.81 mmol), water (two or three drops), and DMSO (3 mL) was refluxed at 180°C for 20 hours. The mixture was allowed to cool to room temperature. Then, saturated brine was added thereto, and the product was extracted with diethyl ether four times and washed with saturated brine twice. The organic layer was dried over sodium sulfate, filtered, and concentrated under reduced pressure. The residue was evaporated (6 Torr, 153°C) to obtain 2,2-dimethyl-5-carboethoxy-1,3-dioxane (181 mg, 64%) as a pale yellow oil.

¹H-NMR of 2,2-dimethyl-5-carboethoxy-1,3-dioxane was as follows.

¹H-NMR (500 MHz, CDCl₃) δ: 4.17 (q, J = 21.8 Hz, 2H), 4.04 (d, J = 20.0 Hz, 4H), 2.80 (m, J = 28.1 Hz, 1H), 1.45 (s, 3H), 1.42 (s, 3H), 1.27 (d, J = 14.3 Hz, 3H)

### (Step 19-step 20)

Step 19 can be carried out by hydrolyzing 2,2-dimethyl-5-carboethoxy-1,3-dioxane with lithium hydroxide in a THF solvent. Step 20 can be carried out by reacting the product obtained in step 19 with oxalyl chloride in a methylene chloride solvent at room temperature.

### (Step 21)

A solution of 2,2-dimethyl-5-carboethoxy-1,3-dioxane (207 mg, 1.10 mmol) in THF (2 mL) was added dropwise to a solution of lithium aluminum hydroxide (88.3 mg, 2,33 mmol) in THF (2 mL) in an argon atmosphere, and the mixed solution was then heated to reflux for 20 hours. The mixture was allowed to cool. Then, a 6 M aqueous sodium hydroxide solution (10 mL) was added thereto, and the product was extracted with ethyl acetate four times. This organic layer was dried over sodium sulfate, then filtered, and concentrated under reduced pressure to obtain 2,2-dimethyl-1,3-dioxane-5-methanol (80 mg, 50%) as a pale yellow oil.

¹H-NMR of 2,2-dimethyl-1,3-dioxane-5-methanol was as follows.

¹H NMR (500MHz, CDCl₃) δ: 4.03 (dd, J = 12.0, 4.0 Hz, 2H), 3.77-3.80 (m, 4H), 1.83 (m, 1H), 1.66 (t, J = 5.1 Hz, 1H), 1.45 (s, 3H), 1.41 (s, 3H)

### (Step 22)

2,2-Dimethyl-1,3-dioxane-5-methanol (59 mg, 0.40 mmol), dichloromethane (2 mL), and triethylamine (0.1 mL) were mixed and cooled to 0°C. Tosyl chloride (102 mg, 0.7 mmol) was added thereto, and the mixed reaction solution was stirred at room temperature for 1 hour. Then, the reaction solution was washed with saturated ammonium chloride salt twice and with saturated brine once, and the product was extracted with dichloromethane. The organic layer was dried over sodium sulfate and concentrated to obtain intermediate V' (L = p-toluenesulfonyl) (82 mg, 90%) as a light brown oil.

¹H-NMR of the intermediate V' (L = p-toluenesulfonyl) was as follows.

¹H-NMR (500 MHz, CDCl₃) δ: 7.79-7.81 (d, J = 8.0 Hz, 2H), 7.36 (d, J = 8.0 Hz, 2H), 4.17 (d, J = 6.9 Hz, 2H), 3.97 (dd, J = 12.3, 3.7 Hz, 2H), 3.68 (dd, J = 12.0, 4.6 Hz, 2H), 2.45 (s, 3H), 1.95 (m, J = 22.3 Hz, 1H), 1.40 (s, 3H), 1.30 (s, 3H)

### <Synthesis of intermediates VI' and VI>

Next, intermediate VI' is synthesized from the intermediate V' according to the scheme given below. Also, intermediate VI is synthesized from the intermediate V according to the following scheme.

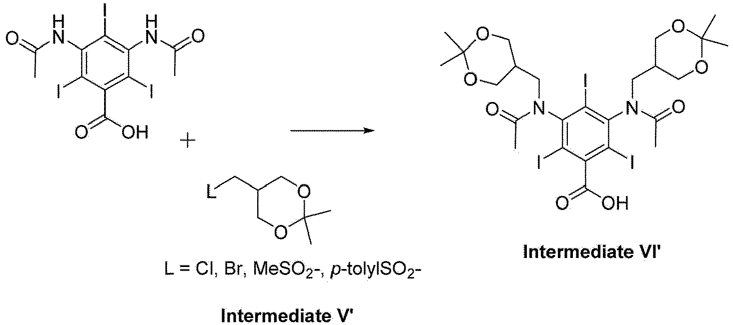

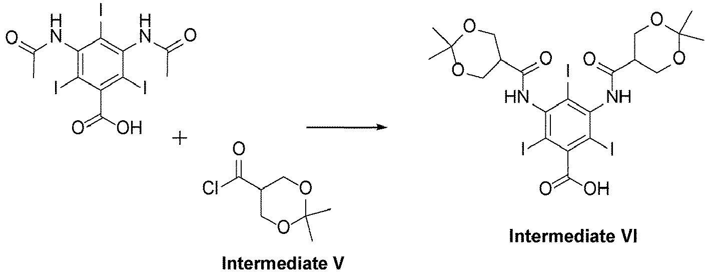

Specifically, the synthesis of the intermediate VI' can be carried out by hydrolyzing commercially available diatrizoic acid with hydrochloric acid, followed by reaction with the intermediate V'. The synthesis of the intermediate VI can be carried out by treating commercially available diatrizoic acid with a base, followed by reaction with the intermediate V.

### <Synthesis of PK-1 and PK-2>

The following PK-1 and PK-2 can be synthesized using the azide obtained in step 2 of the method for synthesizing the intermediate II in Example 1, and the intermediate VI' or VI.

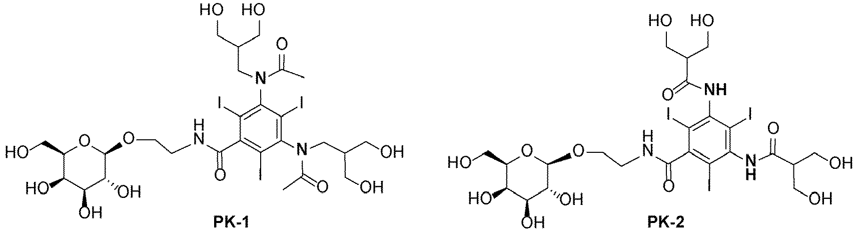

Specifically, PK-1 can be synthesized according to the scheme given below. The azide obtained in step 2 of the method for synthesizing the intermediate II is aminated by the method of step 4 of the method for synthesizing the intermediate II in Example 1. The obtained amine is coupled with the intermediate VI' using a peptide bond formation reagent such as DCC or EDC and then deprotected by base treatment and acid treatment to obtain PK-1.

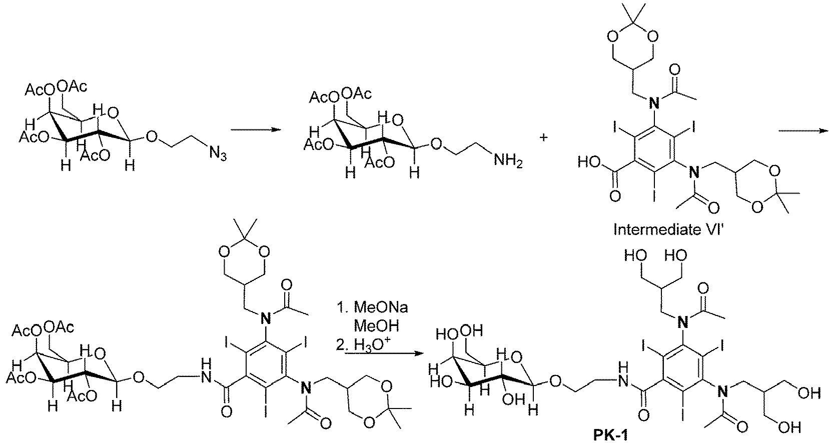

PK-2 can be synthesized from the intermediate VI according to the following scheme in the same manner as above.

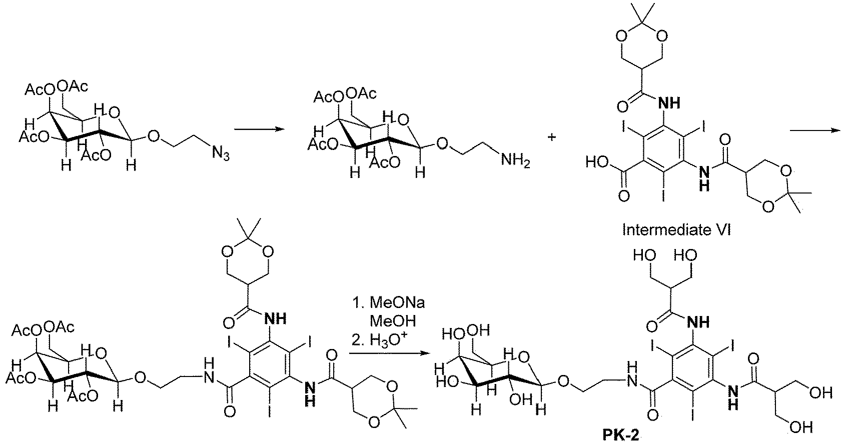

### [Example 9] Synthesis of KOG-1

For the synthesis of KOG-1, intermediate II'' was first synthesized.

### <Synthesis of intermediate II''>

The intermediate II'' was synthesized according to the following scheme.

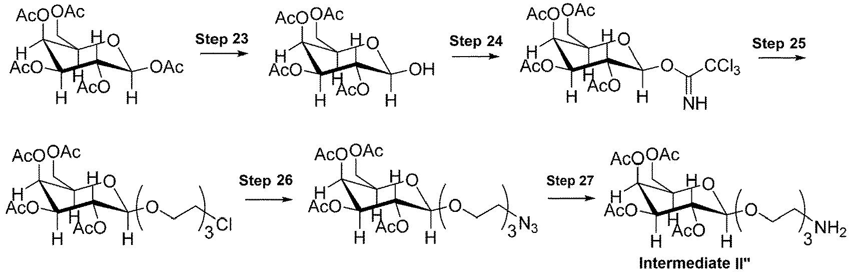

### (Step 23)

Commercially available penta-O-acetyl-β-D-galactopyranose (manufactured by Tokyo Chemical Industry Co., Ltd., 5.04 g, 12.9 mmol) and benzylamine (2.8 mL, 25.6 mmol) were dissolved in dehydrated THF (50 mL) in an argon atmosphere. The solution was stirred at room temperature for 23 hours. Then, the solvent was distilled off under reduced pressure, and ethyl acetate was added to the residue. This organic layer was washed with 2.0 M hydrochloric acid, dried over sodium sulfate, and filtered, and the solvent was then distilled off under reduced pressure. The residue was purified by silica gel chromatography (n-hexane: ethyl acetate = 1: 1) to obtain 2,3,4,6-tetra-O-acetyl-β-D-galactopyranose (4.5 g, quantitative) as a colorless oil (Rf = 0.36, n-hexane: ethyl acetate = 1:1).

¹H-NMR of 2,3,4,6-tetra-0-acetyl-β-D-galactopyranose was as follows.

¹H-NMR (500 MHz, CDCl₃) δ: 5.52 (t, J = 3.4 Hz, 1H), 5.48 (t, J = 1.7 Hz, 1H), 5.42 (dd, J = 10.9, 3.4 Hz, 1H), 5.16 (dd, J = 10.9, 3.4 Hz, 1H), 4.48 (t, J = 6.6 Hz, 1H), 4.07-4.16 (m, 2H), 3.48 (br, 1H), 2.16 (s, 3H), 2.11 (s, 3H), 2.06 (s, 3H), 2.00 (s, 3H)

### (Step 24)

2,3,4,6-Tetra-O-acetyl-β-D-galactopyranose (3.87 g, 11.1 mmol) and trichloroacetonitrile (5.50 mL, 55.4 mmol) were dissolved in dehydrated dichloromethane (35 mL) in an argon atmosphere. 1,8-Diazabicyclo[5.4.0]undec-7-ene (DBU, 0.34 mL, 2.23 mmol) was added dropwise to the solution under ice cooling, and the mixture was stirred for 2 hours. Then, the solvent was distilled off under reduced pressure, and ethyl acetate was added to the residue. This organic layer was washed with water and saturated brine, dried over anhydrous sodium sulfate, then filtered, and concentrated under reduced pressure to obtain crude (1-(2,2,2-trichloroethanimidate)-2,3,4,6-tetraacetate-β-D-galactopyranoside (Rf = 0.40, n-hexane: ethyl acetate = 2:1).

¹H-NMR of (1-(2,2,2-trichloroethanimidate)-2,3,4,6-tetraacetate-β-D-galactopyranoside was as follows.

¹H-NMR (400 MHz, CDCl₃) δ: 8.67 (s, 1H), 6.60 (d, J = 3.5 Hz, 1H), 5.56 (d, J = 2.0 Hz, 1H), 5.35-5.45 (m, 2H), 4.44 (t, J = 6.5 Hz, 1H), 4.06-4.19 (m, 2H), 2.17 (s, 3H), 2.02-2.05 (m, 9H)

### (Step 25)

The crude (1-(2,2,2-trichloroethanimidate)-2,3,4,6-tetraacetate-β-D-galactopyranoside and 2-[2-(2-chloroethoxy)ethoxy]ethanol (4.6 mL, 32.2 mmol) were dissolved in dehydrated dichloromethane (90 mL) in an argon atmosphere. Boron trifluoride-diethyl etherate (10.6 mL, 86.2 mmol) was added dropwise to the solution under ice cooling, and the mixture was stirred for 1 hour under ice cooling and then stirred overnight at room temperature while shielded from light. Water was added to the reaction solution, and the solvent was distilled off under reduced pressure. Ethyl acetate was added to the residue, and the mixture was washed with water, a saturated sodium bicarbonate solution, and saturated brine in order, dried over anhydrous sodium sulfate, and then filtered. The filtrate was concentrated under reduced pressure and purified by silica gel column chromatography (n-hexane: ethyl acetate = 4: 1) to obtain crude (2R,3S,4S,5R,6R)-2-(acetoxymethyl)-6-(2-(2-(2-chloroethoxy)ethoxy)ethoxy)tetrahydro-2H-pyran-3,4,5-triyl triacetate as a yellow oil.

¹H-NMR and results of mass spectrometry of the obtained chloride were as follows.

¹H-NMR (500 MHz, CDCl₃) δ: 5.38 (q, J = 1.4 Hz, 1H), 5.20 (dd, J = 10.9, 8.0 Hz, 1H), 5.01 (dd, J = 10.3, 3.4 Hz, 1H), 4.56 (d, J = 8.0 Hz, 1H), 4.19-4.07 (m, 3H), 3.78-3.61 (m, 12H), 2.14 (s, 3H), 2.05 (d, J = 6.9 Hz, 6H), 1.98 (s, 3H); LRMS (ESI) m/z [M+Na]⁺ calcd for C₂₀H₃₁C₁₃NaO₁₂: 521; found 521.

### (Step 26)

The obtained chloride and sodium azide (709 mg, 10.9 mmol) were dissolved in dehydrated DMSO (32 mL) in an argon atmosphere, and the solution was stirred overnight at 80°C. Ethyl acetate was added to the reaction solution, and the organic layer was washed with water, a saturated aqueous solution of sodium bicarbonate, and saturated brine in order. The organic layer was dried over anhydrous sodium sulfate, then filtered, and concentrated under reduced pressure. The residue was purified by silica gel column chromatography (n-hexane: ethyl acetate = 2: 3) to obtain (2R,3S,4S,5R,6R)-2-(acetoxymethyl)-6-(2-(2-(2-azidoethoxy)ethoxy)ethoxy)tetrahydro-2H-pyran-3,4,5-triyl triacetate (94 mg, 44% from 3 steps) as a yellow oil.

¹H-NMR and results of mass spectrometry of the obtained azide were as follows.

¹H-NMR (500 MHz, CDCl₃) δ: 5.46 (d, J = 2.9 Hz, 1H), 5.37 (dd, J = 10.9, 3.4 Hz, 1H), 5.17 (d, J = 3.4 Hz, 1H), 5.13 (dd, J = 10.9, 3.4 Hz, 1H), 4.33-4.17 (m, 2H), 4.16-4.04 (m, 2H), 3.72-3.61 (m, 9H), 3.40 (t, J = 4.9 Hz, 2H), 2.14 (s, 3H), 2.08 (s, 3H), 2.04 (s, 3H), 1.99 (s, 3H); LRMS (ESI) m/z [M+Na]⁺ calcd for C₂₀H₃₁N₃NaO₁₂: 528; found 528.

### (Step 27)

The obtained azide (330 mg, 0.65 mmol), 10% palladium carbon (30 mg) and dehydrated methanol (8 mL) were mixed and stirred at room temperature for 24 hours in a hydrogen atmosphere. The reaction solution was filtered, and the solvent was distilled off under reduced pressure to obtain intermediate II'' which was (2R,3S,4S,5R,6R)-2-(acetoxymethyl)-6-(2-(2-(2-aminoethoxy)ethoxy)ethoxy)tetrahydro-2H-pyran-3,4,5-triyl triacetate (170 mg, 54%) as a brown oil.

¹H-NMR of the intermediate II'' was as follows. ¹H-NMR (500 MHz, CDCl₃) δ: 5.39 (d, J = 3.4 Hz, 1H), 5.21 (dd, J = 10.3, 8.0 Hz, 1H), 5.02 (dd, J = 10.3, 3.4 Hz, 1H), 4.58 (d, J = 8.0 Hz, 1H), 4.20-4.10 (m, 2H), 3.99-3.90 (m, 2H), 3.79-3.73 (m, 1H), 3.69-3.59 (m, 8H), 3.52 (t, J = 5.2 Hz, 2H), 2.88 (t, J = 5.2 Hz, 2H), 2.15 (s, 3H), 2.06 (d, J = 6.3 Hz, 6H), 1.99 (s, 3H)

### <Synthesis of KOG-1>

KOG-1 can be synthesized from intermediate VII according to the scheme given below. The intermediate VII was synthesized from the intermediate II'' and the intermediate IV.

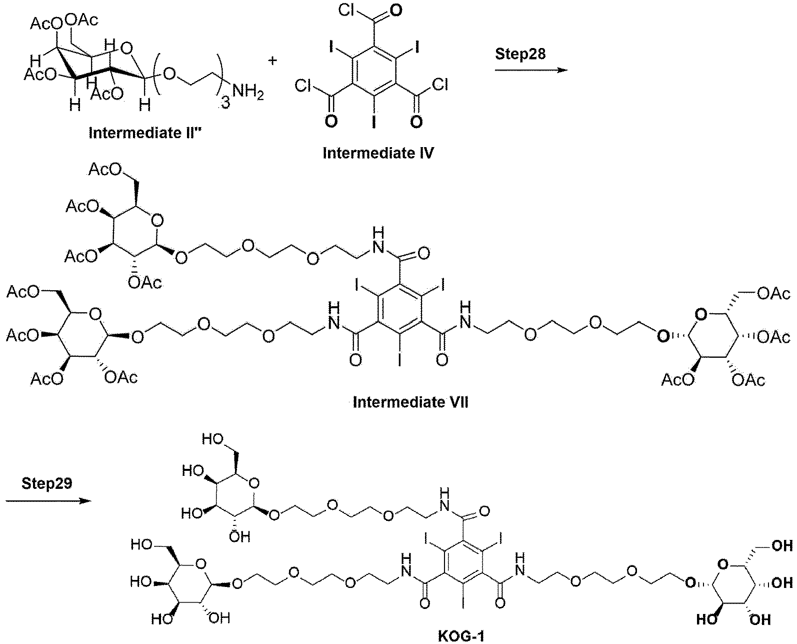

### (Step 28)

The intermediate II'' (800 mg, 1.68 mmol) and the intermediate IV (308 mg, 0.48 mmol) were dissolved in dehydrated DMF (10 mL) in an argon atmosphere, and triethylamine (810 µL) was then gradually added dropwise to the solution. This mixed reaction solution was stirred at room temperature for 23 hours, and the solvent was then distilled off under reduced pressure. The residue was purified by silica gel column chromatography (chloroform: methanol = 9: 1) and then further purified by size-exclusion chromatography (chloroform) to obtain intermediate VII which was a precursor of KOG-1.

Results of mass spectrometry of the intermediate VII were as follows.

LRMS (ESI) m/z [M+Na]⁺ calcd for C₆₉H₉₆N₃NaO₃₉: 1994; found 1994.

### (Step 29)

The intermediate VII is dissolved in dehydrated methanol. A 1 M solution of methanol methoxide in methanol is added to the solution until pH became 9, followed by stirring at room temperature for 3 hours. The reaction mixture is neutralized by the addition of an ion-exchange resin (Amberlite H+) and filtered, and the filtrate can be concentrated to obtain KOG-1.

### [Example 10] Synthesis of MEG-4

MEG-4 was synthesized according to the following scheme which was different from that of Example 5.

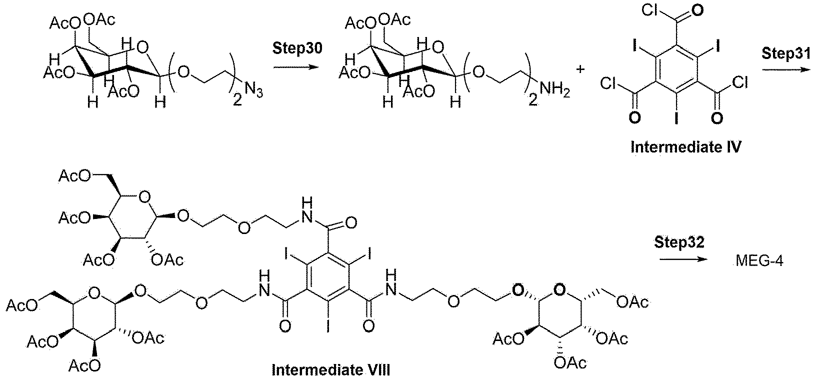

### (Step 30)

2-(2-Azidoethoxy)ethyl-2,3,4,6-tetraacetate-β-D-galactopyranoside (33.7 mg, 0.731 mmol) which was the azide obtained in step 2 of the method for synthesizing the intermediate II in Example 1, 10% palladium carbon (3.7 mg) and dehydrated methanol/isopropanol (1.0 mL/1.5 mL) were mixed and stirred at room temperature for 2 hours in a hydrogen atmosphere. The reaction solution was filtered, and the solvent was distilled off under reduced pressure to obtain crude (2R,3S,4S,5R,6R)-2-(acetoxymethyl)-6-(2-(2-aminoethoxy)ethoxy)tetrahydro-2H-pyran-3,4,5-triyl triacetate (31.6 mg, 99%) as a brown oil (Rf = 0.13, chloroform: methanol = 9 :1).

¹H-NMR of the obtained amine was as follow.

¹H-NMR (500 MHz, CDCl₃) δ: 5.40 (d, J = 2.9 Hz, 1H), 5.18 (dd, J = 10.3, 8.0 Hz, 1H), 5.08 (dd, J = 10.9, 3.4 Hz, 1H), 4.60 (d, J = 8.0 Hz, 1H), 4.21 (q, J = 5.9 Hz, 1H), 4.13 (dd, J = 11.5, 6.9 Hz, 1H), 3.97-4.02 (m, 2H), 3.68-3.76 (m, 5H), 3.17 (s, 2H), 2.18 (s, 3H), 2.10 (s, 3H), 2.06 (s, 3H), 1.99 (s, 3H)

### (Step 31)

The obtained amine (product synthesized from 1.90 mmol of the azide) and the intermediate IV (302 mg, 0.471 mmol) were dissolved in dehydrated DMF (10 mL) in an argon atmosphere, and triethylamine (660 µL) was then gradually added dropwise to the solution. This mixed reaction solution was stirred at room temperature for 45 hours, and the solvent was then distilled off under reduced pressure. The residue was purified by silica gel column chromatography (chloroform: methanol = 100:1 to 1:9) and then further purified by size-exclusion chromatography (chloroform) to obtain intermediate VIII (42.2 mg, 5%) which was a precursor of MEG-4 as a yellow oil.

¹H-NMR and results of mass spectrometry of the intermediate VIII were as follows.

¹H-NMR (500 MHz, CDCl₃) δ: 5.38 (d, J = 3.2 Hz, 3H), 5.14-5.18 (m, 3H), 4.98-5.01 (m, 3H), 4.50-4.53 (m, 3H), 4.11-4.15 (m, 6H), 3.97 (s, 6H), 3.63-3.75 (m, 21H), 2.15 (s, 9H), 2.02-2.05 (m, 18H), 1.98 (s, 9H); HRMS (ESI) m/z [M+Na]⁺ calcd for C₆₃H₈₄I₃N₃O₃₆: 1862.1694; found 1862.1686.

### (Step 32)

The intermediate VIII (5.2 mg, 2.83 µmol) was dissolved in dehydrated methanol (1.0 mL) in an argon atmosphere, and a 1 M solution of methanol methoxide in methanol was added to the solution until pH became 9, followed by stirring at room temperature for 3 hours. Then, the reaction solution was neutralized by the addition of an ion-exchange resin (Amberlite H+) and filtered, and the filtrate was concentrated to obtain MEG-4.

### [Test Example 1] Uptake inhibition test of compound

RYO-1, MEG-1, and MEG-2 prepared in Examples were subjected to an uptake inhibition test, in which the uptake is performed by an asialoglycoprotein receptor specifically expressed in hepatocytes, using a liver cancer cell line HepG2 in accordance with J Gastroenterol 1998, 33, 855-859. Specifically, the test is based on the following method.

First, orosomucoid (Sigma-Aldrich Co., LLC) was converted to an asialo compound through reaction with immobilized neuraminidase (Sigma-Aldrich Co., LLC) at 37°C for 12 hours. This asialo-orosomucoid (ASOR) was labeled with radioactive iodine (125I) by use of the chloramine-T method to prepare I-ASOR.

The obtained I-ASOR was prepared as a 6 µg/mL solution in PBS and reacted with HepG2 cells washed with ice-cold MEM (GIBCO) in 6 wells at 4°C for 30 minutes. The cells were thoroughly washed and then lysed using 0.1 N NaOH, and radiation dose "A" taken up into the cells was measured (control sample). Similar measurement was performed by the addition of I-ASOR as well as MEG-1, MEG-2, or RYO-1 at 100 µg/mL to measure radiation dose "A" when each compound was added.

Next, the background of nonspecific binding of I-ASOR was measured as follows in order to eliminate the influence of the nonspecific binding of I-ASOR to the cells. Specifically, I-ASOR (6 µg/mL) and a 100-fold concentration (600 µg/mL) of nonlabelled ASOR were mixed and reacted with HepG2 in the same manner as above. Then, the obtained cells were washed and lysed, and radiation dose "B" was measured. The radiation dose "B" as the background was commonly used in all of the control sample and the sample supplemented with each compound.

Next, "A - B" was calculated as to each sample to calculate I-ASOR specifically taken up into HepG2. The experiment was conducted in triplicate to calculate standard deviation.

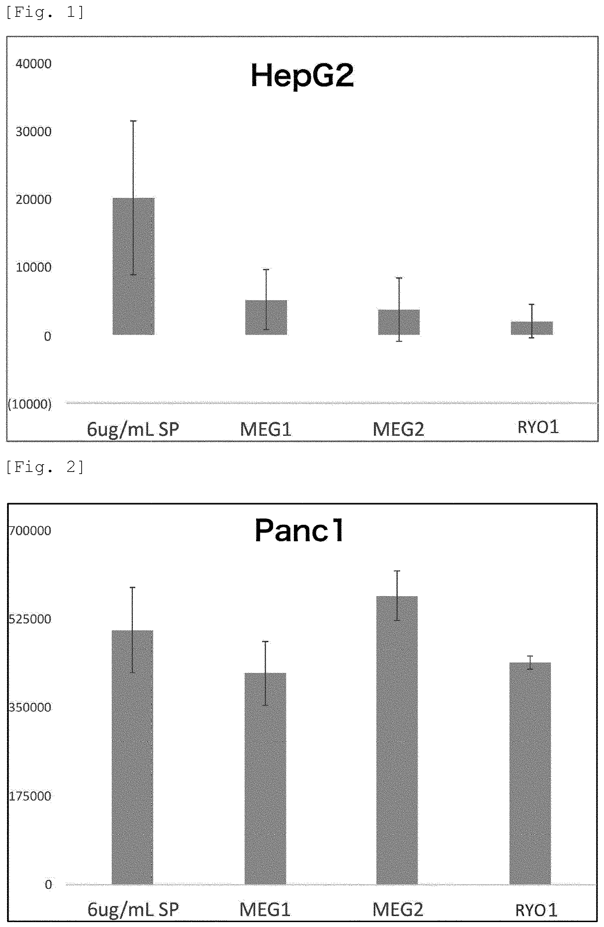

In HepG2, all of RYO-1, MEG-1, and MEG-2 were found to competitively inhibit the uptake of the ligand. Figure 1 shows the results of the uptake inhibition test in HepG2. In Figure 1, the label "6 µg/mL SP" depicts the control sample.

A similar experiment was conducted using a pancreatic cancer cell line Panc-1 having no asialoglycoprotein receptor. Specifically, the test is based on the following method.

I-ASOR labeled with radioactive iodine (125I) in the same manner as above was prepared as a 6 µg/mL solution in PBS and reacted with Panc-1 cells washed with ice-cold MEM (GIBCO) in 6 wells at 4°C for 30 minutes. The cells were thoroughly washed and then lysed using 0.1 N NaOH, and radiation dose "A" taken up into the cells was measured (control sample). Similar measurement was performed by the addition of I-ASOR as well as MEG-1, MEG-2, or RYO-1 at 100 µg/mL to measure radiation dose "A" when each compound was added.

Next, the background of nonspecific binding of I-ASOR was measured as follows in order to eliminate the influence of the nonspecific binding of I-ASOR to the cells. Specifically, I-ASOR (6 µg/mL) and a 100-fold concentration (600 µg/mL) of nonlabelled ASOR were mixed and reacted with Panc-1 in the same manner as above. Then, the obtained cells were washed and lysed, and radiation dose "B" was measured. The radiation dose "B" as the background was commonly used in all of the control sample and the sample supplemented with each compound.

Next, "A - B" was calculated as to each sample to calculate I-ASOR specifically taken up into Panc-1. The experiment was conducted in triplicate to calculate standard deviation.

In Panc-1, none of RYO-1, MEG-1, and MEG-2 inhibited the uptake of the ligand. Figure 2 shows the results of the uptake inhibition test in Panc-1.

These results demonstrated that the compound of the present invention is specifically taken up into hepatocytes.

### [Test Example 2] Comparison of uptake activity by asialoglycoprotein receptor among compounds

Uptake activity by an asialoglycoprotein receptor was compared among MEG-2, MEG-4, RYO-1, and RYO-2 by use of the same method as in Test Example 1.

A PBS solution containing 0.4 µg/mL I-ASOR obtained in the same manner as in Test Example 1 and 1.0 mM/mL MEG-2, MEG-4, RYO-1, or RYO-2 was prepared and reacted with HepG2 washed with ice-cold MEM (GIBCO) in 6 wells at 37°C for 30 minutes. The cells were thoroughly washed and then lysed using 0.1 N NaOH, and radiation dose "A" taken up into HepG2 was measured.

An experiment was conducted as a positive control in which a PBS solution containing 0.4 µg/mL I-ASOR and 1000 µg/mL nonlabelled ASOR was prepared and reacted with HepG2 washed with ice-cold MEM (GIBCO) in 6 wells at 4°C for 30 minutes.

Next, the background of nonspecific binding of I-ASOR was measured as follows in order to eliminate the influence of the nonspecific binding of I-ASOR to the cells. Specifically, I-ASOR (0.4 µg/mL) and a 100-fold concentration (40 µg/mL) of nonlabelled ASOR were mixed and reacted with HepG2 in the same manner as above. Then, the obtained cells were washed and lysed, and radiation dose "B" was measured. The radiation dose "B" as the background was commonly used in all of the control sample, the positive control sample, and the sample supplemented with each compound.

Next, "A - B" was calculated as to each sample to calculate I-ASOR specifically taken up into HepG2. The experiment was conducted in triplicate to calculate standard deviation.

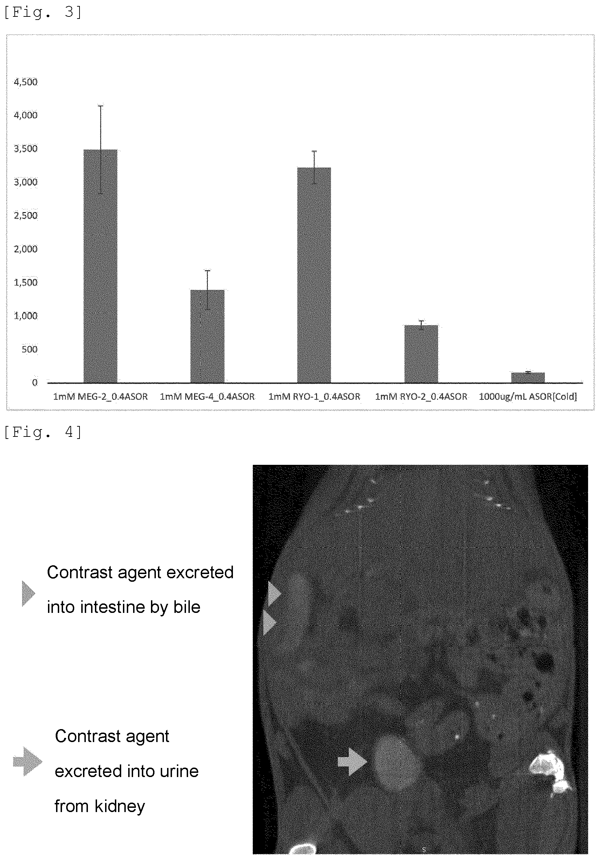

All of MEG-2, MEG-4, RYO-1, and RYO-2 competitively inhibited the uptake of the ligand in HepG2. Such inhibitory ability was found to be higher in MEG-4 than in MEG-2 and to be higher in RYO-2 than in RYO-1. The results are shown in Figure 3. These results suggested that a compound having a larger number of atomic groups that bind to an asialoglycoprotein receptor has higher uptake activity by the asialoglycoprotein receptor.

### [Test Example 3] Contrast experiment

A 9-week-old male ICR mouse purchased from CLEA Japan, Inc. was anesthetized by isoflurane inhalation. While the respiratory condition was monitored with DELPet µCT100 application software (DELBio, Inc.), MEG-1 was gradually administered by injection from the tail vein. The dose of MEG-1 was set to 52 mg/animal.

CT images were taken before administration, during administration, 15 minutes after administration and 1 hour after administration. Image data from the chest to the lower abdomen was reconstituted at a pixel size of 45 µm and converted to dicom data. Further, the images were analyzed with VivoQuant software (inviCRO. LLC). As a result, a liquid that exhibited high absorption, which was not observed before administration, was confirmed to appear over time in the intestine and in the urinary bladder.

Figure 4 shows the image taken 1 hour after administration.

A similar test was conducted using MEG-2, MEG-4, or RYO-2 instead of MEG-1. As a result, a liquid that exhibited high absorption, which was not observed before administration, was confirmed to appear over time in the intestine and in the urinary bladder, in all the cases.

The results described above including the results of Figure 4 demonstrated that a compound having a site that is recognized by an asialoglycoprotein receptor has a function of excretion into bile and is excreted through two systems of excretion pathways. Specifically, the compound enables adverse reactions to be alleviated and the liver to be diagnosed by visualizing hepatocyte functions.

### Industrial Applicability

The present invention has industrial applicability to diagnostic imaging and the like.

## Claims

1. A compound represented by the formula (1) or a pharmaceutically acceptable salt thereof: 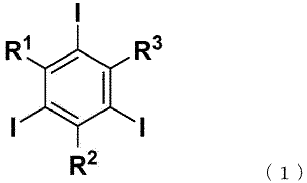 wherein
R¹ to R³ are each independently
an amino group represented by -NR^{x}R^{y} wherein R^{x} and R^{y} each independently represent a hydrogen atom, a C1 to C6 hydrocarbon group optionally having a substituent, or a C2 to C7 acyl group optionally having a substituent; or
an amide group represented by -C(=O)NR^{z}R^{w} wherein R^{z} and R^{w} each independently represent a hydrogen atom or a C1 to C6 hydrocarbon group optionally having a substituent; or
a group represented by the formula (2): 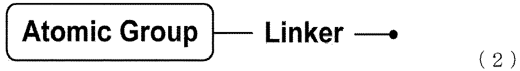 wherein
Atomic Group is an atomic group that binds to an asialoglycoprotein receptor, and
Linker is an arbitrary linker, and
at least one of R¹ to R³ is the group represented by the formula (2).

2. The compound according to claim 1 or a pharmaceutically acceptable salt thereof, wherein
the Atomic Group is a sugar residue that binds to an asialoglycoprotein receptor.

3. The compound according to claim 1 or 2 or a pharmaceutically acceptable salt thereof, wherein
the Atomic Group is a group derived from galactose, N-acetylgalactosamine, N-trifluoroacetylgalactosamine, or galactose-N-acetylglucosamine.

4. The compound according to any one of claims 1 to 3 or a pharmaceutically acceptable salt thereof, wherein
the group represented by the formula (2) is a group represented by the following formula (2-1), (2-2), (2-3), or (2-4): 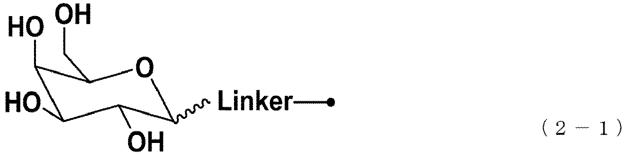 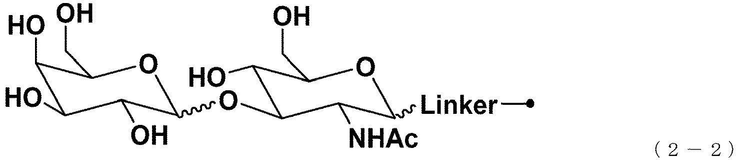 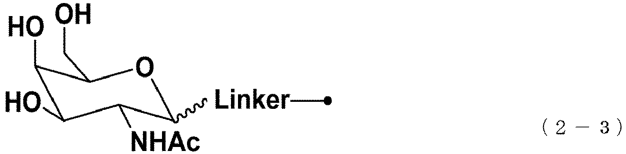 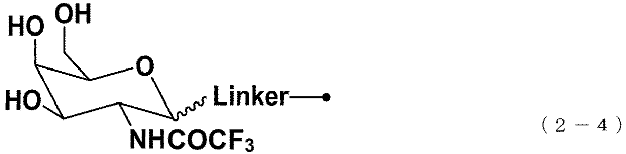 wherein Linker is an arbitrary linker.

5. The compound according to any one of claims 1 to 4 or a pharmaceutically acceptable salt thereof, wherein
the linker is a hydrocarbon chain optionally having a substituent, and
one or both of the two ends of the hydrocarbon chain optionally have a heteroatom, an amide bond, an ester bond, a carbonyl bond, or an aromatic heterocycle.

6. The compound according to claim 5 or a pharmaceutically acceptable salt thereof, wherein
the hydrocarbon chain is an alkylene chain optionally having a substituent, or a hydrocarbon chain formed by bonding two or more alkylene chains optionally having a substituent via at least one selected from the group consisting of a heteroatom, an amide bond, an ester bond, a carbonyl bond, and an aromatic heterocycle.

7. The compound according to any one of claims 1 to 6 or a pharmaceutically acceptable salt thereof, wherein
the linker is represented by the following structure: 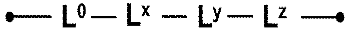 wherein
L° is -O- or -NHC(=O)-,
L^{x} is represented by the following structure: 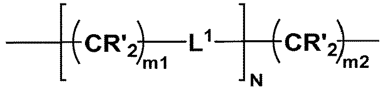
L^{y} represents a single bond or is represented by the following structure: 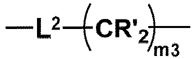 and
L^{z} is represented by the following structure: 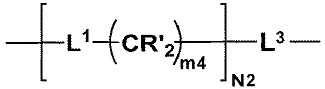 wherein
each R' is independently one selected from a hydrogen atom, a C1 to C3 alkyl group optionally having a substituent, and a hydroxy group,
each L¹ is independently one selected from an ether bond (-O-), a thioether bond (-S-), an amine bond (-NH-), an amide bond, an ester bond, and a carbonyl bond,
L² is an amide bond, or a divalent group derived from an aromatic heterocycle,
L³ is one selected from -OCH₂-, -NHCH₂-, -C(=O)NH-, - C(=O)NHCH₂-, and -NHC(=O)-,
m1, m2, and m4 are each independently an integer of 1 or larger,
m3 is an integer of 0 or larger, and
N and N2 are each independently an integer of 0 or larger.

8. The compound according to claim 7 or a pharmaceutically acceptable salt thereof, wherein
L^{x} is any of the following structures: 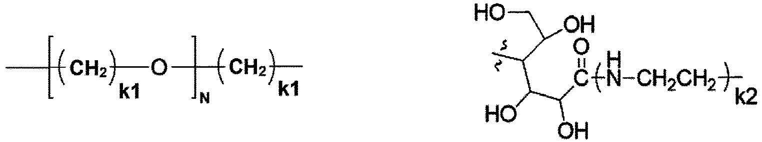
L^{y} is a single bond or any of the following structures: 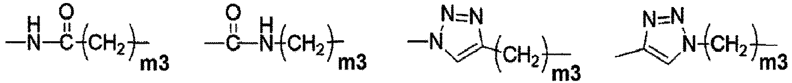 and
L^{z} is any of the following structures: 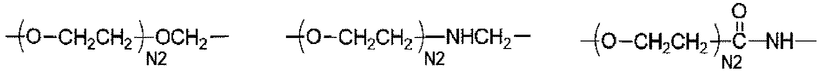 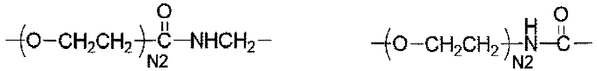 wherein
each k1 is independently an integer of 1 or larger and 3 or smaller,
k2 is 0 or 1,
m3 is an integer of 0 or larger, and
N and N2 are each independently an integer of 0 or larger.

9. The compound according to claim 7 or a pharmaceutically acceptable salt thereof, wherein
the linker is represented by the following structure: 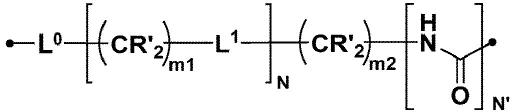 wherein
each R' is independently one selected from a hydrogen atom, a C1 to C3 alkyl group optionally having a substituent, and a hydroxy group,
L° is -O- or -NHC(=O)-,
each L¹ is independently one selected from an ether bond (-O-), a thioether bond (-S-), an amine bond (-NH-), an amide bond, an ester bond, and a carbonyl bond,
m1 and m2 are each independently an integer of 1 or larger,
N is an integer of 0 or larger, and
N' is 0 or 1.

10. The compound according to claim 9 or a pharmaceutically acceptable salt thereof, wherein
the linker is represented by any of the following structures: 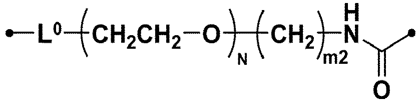 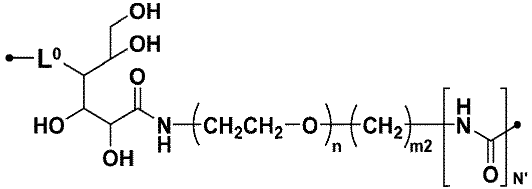 wherein
L° is -O- or -NHC(=O)-,
N is an integer of 0 or larger,
n is an integer of 0 or larger,
m2 is an integer of 1 or larger, and
N' is 0 or 1.

11. The compound according to any one of claims 1 to 10 or a pharmaceutically acceptable salt thereof, wherein
the amino group is represented by any of the following structures: 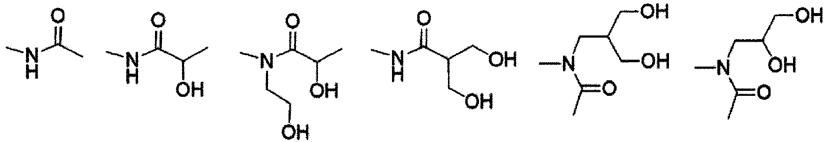 and
the amide group is represented by any of the following structures:

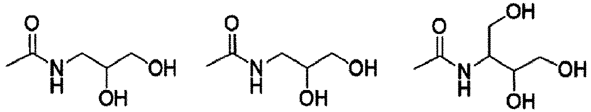

12. The compound according to any one of claims 1 to 11 or a pharmaceutically acceptable salt thereof, wherein
the amino group is an acetylamino group.

13. The compound according to any one of claims 1 to 12 or a pharmaceutically acceptable salt thereof, wherein
in the formula (1), all of R¹ to R³ are groups represented by the formula (2).

14. A compound represented by any of the following structures or a pharmaceutically acceptable salt thereof:

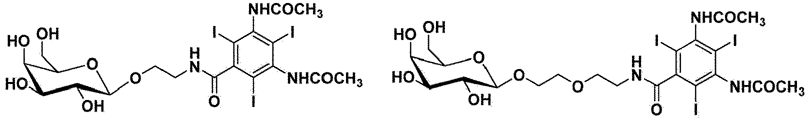

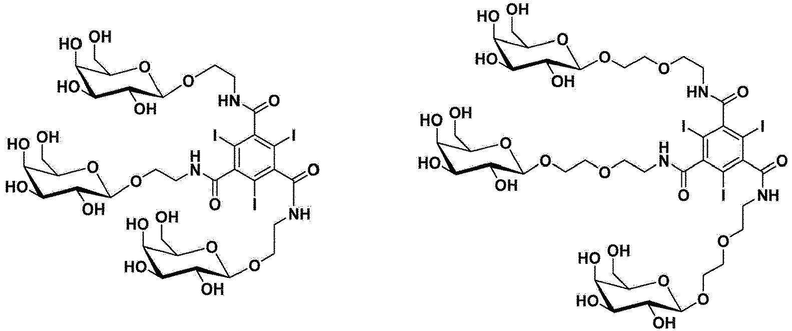

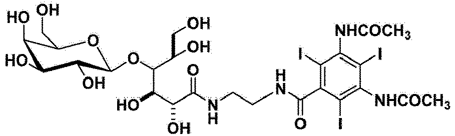

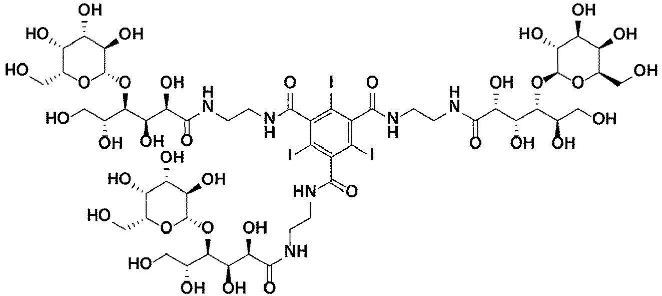

15. A compound represented by the following structure or a pharmaceutically acceptable salt thereof:

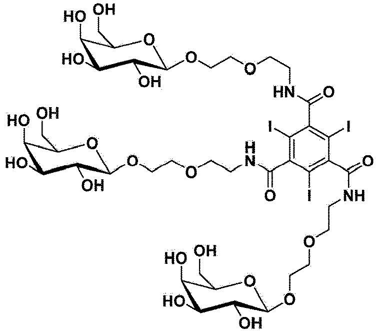

16. A compound represented by the following structure or a pharmaceutically acceptable salt thereof:

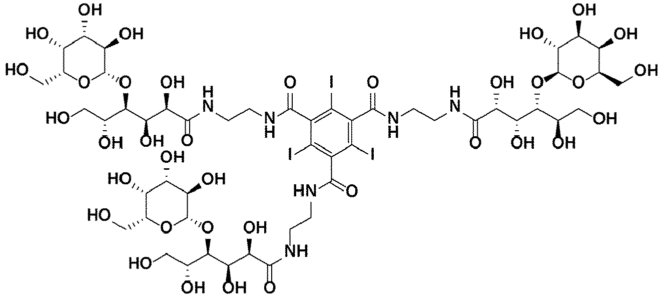

17. A contrast agent comprising a compound according to any one of claims 1 to 16 or a pharmaceutically acceptable salt thereof.

18. A method for producing a compound according to any one of claims 1 to 16 or a pharmaceutically acceptable salt thereof, comprising the step of
reacting a reaction substrate constituted by an atomic group moiety that binds to an asialoglycoprotein receptor and a linker moiety with a reaction substrate of a nonionic iodine contrast agent moiety.
